# EUROPÄISCHE PATENTANMELDUNG

(11) **EP 2 105 120 A1**
(43) Veröffentlichungstag der Anmeldung: **30.09.2009**
(21) Anmeldenummer: 08153269.9
(22) Anmeldetag: 26.03.2008
(51) Int. Cl.: A61K 8/02, A61K 8/87, A61Q 19/00

(54) **Kosmetische Zusammensetzungen zum Auftrag auf die Haut**

(71) Anmelder: Bayer MaterialScience AG, 51368 Leverkusen (DE)
(72) Erfinder: Die Erfindernennung liegt noch nicht vor

(57) **Zusammenfassung**

Die Erfindung betrifft eine kosmetische Zusammensetzung zum Auftrag auf die Haut, ein Verfahren zu ihrer Herstellung sowie ihre Verwendung.

## Beschreibung

Die Erfindung betrifft kosmetische Zusammensetzungen zum Auftrag auf die Haut, Verfahren zu ihrer Herstellung sowie ihre Verwendung insbesondere als Hautreinigungszusammensetzungen, wie als abziehfähige Reinigungsmasken, insbesondere Gesichtsmasken.

Kosmetische Reinigungsmasken auf Basis wässriger Systeme sind bekannt, weisen jedoch verschiedene Nachteile auf. Insbesondere der notwendige hohe Gehalt an Füllstoffen wie Kaolinen, Tonen, Stärke, Metalloxide wie Zinkoxid und Stärkederivaten führt häufig zu negativen Einflüssen bzgl. der Lagerstabilität bzw. auf die Eigenschaften der Filmbildung des Systems. Die entstandenen Filme werden durch hohe Füllstoffgehalte unregelmäßig und die Maske lässt sich häufig nicht in einem Stück abziehen, wodurch die Anwendung an Effizienz verliert. Auch die oftmals veränderliche toxikologische Einstufung der Füllstoffe, wie beispielsweise des bislang häufig eingesetzten Zinkoxides als "umweltgefährdend", bewirkt einen Nachteil bei kosmetischen Systemen, die auf hohe Konzentrationen derartiger Füllstoffe angewiesen sind.

Die EP-A-1174119 beschreibt die Verwendung von Polyurethandispersionen mit nicht-filmbildenden Füllstoffen, ausgewählt aus Kieselsäuren, Talk, Tonen, Titandioxid, Mikrokugeln, Stärke und modifizierten Stärkeverbindungen. Die EP-A-1093802 beschreibt kosmetische Zusammensetzungen enthaltend Polyurethandispersionen und Kaolin als Füllstoff. Ziel dieser Anmeldungen war es, kosmetische Zusammensetzungen mit einem besonders hohen Gehalt der Füllstoffe bereitzustellen, was durch die Verwendung der Polyurethandispersionen gelingen soll. Als zusätzliche Bausteine der Polyurethane werden verschiedene Sequenzen erwähnt. In den konkreten Beispielen wird insbesondere ein Polyurethan mit einer Polyestersequenz verwendet. Derartige Polyester-Polyurethane weisen jedoch insbesondere vergleichsweise geringe Reißdehnungswerte auf, was ebenfalls zu einer Verschlechterung der Filmbildungseigenschaf ten und der Elastizität der kosmetischen Zusammensetzungen führt. Auch ist Stabilität der im Stand der Technik verwendeten Polyurethandispersionen vergleichsweise gering. Des weiteren führt die Verwendung großer Mengen der genannten Füllstoffe führt zu den oben beschriebenen Nachteilen.

Aufgabe der vorliegenden Erfindung war daher die Bereitstellung von kosmetischen Zusammensetzungen, die diese Nachteile vermeidet. Es wurde nun überraschenderweise gefunden, dass durch den Einsatz spezieller Polyurethandispersionen, enthaltend Sequenzen aus Polyetherbausteinen und/oder Polycarbonatbausteinen und/oder Polyether-Polycarbonat-Bausteinen kosmetische Zusammensetzungen, wie insbesondere abziehbare Haut- bzw. Gesichtsmasken hergestellt werden können, bei denen der Gehalt an notwendigen Füllstoffen reduziert werden oder sogar gänzlich auf die Füllstoffe verzichtet werden kann.

Die Verwendung der erfindungsgemäßen kosmetischen Zusammensetzung ermöglicht einen gleichmäßigen Auftrag auf die Haut, eine schnelle Trocknung darauf, mithin eine schnelle Abziehbarkeit nach dem Auftrag, insbesondere die Abziehbarkeit in einem Stück, verbunden mit einem geringen Schmerzempfinden beim Abziehen, eine sehr gute Reinigungswirkung und eine geringe Gesichtsrötung. Die Nachteile der nicht-filmbildenden Füllstoffe, wie Kaolin, wie eine schlechte Abziehbarkeit in einem Stück werden mittels der speziellen Polyurethane überkommen.

Die vorliegende Erfindung stellt somit kosmetische Zusammensetzungen zum Auftrag auf die Haut bereit, enthaltend ein oder mehrere Polyurethane, wobei genannte Polyurethane mindestens eine Sequenz enthalten, die ausgewählt wird aus der Gruppe, die besteht aus: Polyether-Sequenzen, Polycarbonat-Sequenzen und Polyether-Polycarbonat-Sequenzen.

Polyurethane im Sinne der Erfindung sind polymere Verbindungen, die mindestens zwei Urethan-Gruppen-haltige Wiederholungseinheiten aufweisen: 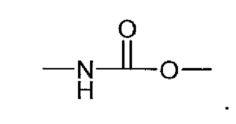 Erfindungsgemäß sind auch solche Polyurethane eingeschlossen, die herstellungsbedingt auch Harnstoff-Gruppen-haltige Wiederholungseinheiten: 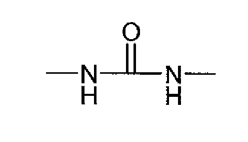 aufweisen, wie sie insbesondere bei der Kettenverlängerung isocyanatterminierter Prepolymere mit Polyaminen gebildet werden.

Die erfindungsgemäßen kosmetischen Zusammensetzungen weisen im Allgemeinen filmbildende Eigenschaften auf. Dies bedeutet, dass sie insbesondere beim Auftrag auf die Haut nach einer gewissen Trockungs- bzw. Einwirkungszeit, wie von beispielsweise mindestens etwa einer Minute einen zusammenhängenden Film bildenden, der im Allgemeinen in einem Stück abgezogen werden kann, was die Anwendung der kosmetischen Zusammensetzungen erheblich erleichtert. Gleichwohl kommt es nicht zu einem Ankleben des Films an der Haut, so dass das Abziehen mit keinerlei Schmerzen oder Reizungen der Haut verbunden ist.

Die in den erfindungsgemäßen kosmetischen Zusammensetzungen verwendeten Polyurethane weisen daher im Allgemeinen eine Reißdehnung von mindestens 500 %, bevorzugt von mindestens 600 %, bevorzugter von mindestens 700 %, noch bevorzugter von mindestens 800 %, noch bevorzugter von mindestens 900 %, ganz besonders bevorzugt von mindestens 1000 %, auf.

In diesem Zusammenhang betrifft die vorliegende Erfindung auch die Verwendung von mindestens einem Polyurethan, welches eine Reißdehnung von mindestens 500 %, bevorzugt von mindestens 600 %, bevorzugter von mindestens 700 %, noch bevorzugter von mindestens 800 %, noch bevorzugter von mindestens 900 %, ganz besonders bevorzugt von mindestens 1000 %, aufweist, zur Herstellung einer kosmetischen Zusammensetzung zum Auftrag auf die Haut, bevorzugt die Gesichtshaut.

Im Rahmen der Erfindung wird die Reißdehnung nach folgendem Verfahren bestimmt.

Die Reißdehnung wird mit einer Reißmaschine der Fa. Zwick, Modell Z 1.0 durchgeführt. Die Durchführung erfolgt gemäß der DIN 53 504 "Prüfung von Kautschuk und Elastomeren". Für diese Prüfung werden Prüfkörper der Größe S 4 ausgestanzt.

Die Prüfgeschwindigkeit ist im Messprogramm voreingestellt und beträgt 400 mm/min. Die Vorkraft beträgt 0,05 N. Pro Film werden jeweils 4 Prüfkörper gerissen.

Die Polyurethandispersion dafür wird mit Borchi Gel ALA auf Streichviskosität (ca. 6000 mPas) eingestellt und wird 500 µm Nassfilm auf Trennpapier VEZ matt mittels WASAG Lineal appliziert. Der Film wird bei 50°C trocknen und anschließend 3 Minuten bei 150°C lagern.

Die Verwendung dieser Polyurethane führt überraschend zu einer Verbesserung der Filmbildungseigenschaften sowie der Elastizität der erfindungsgemäßen kosmetischen Zusammensetzungen bei gleichzeitiger leichter und gleichwohl schmerzfreier Abziehbarkeit.

In den erfindungsgemäßen kosmetischen Zusammensetzungen liegt das Polyurethan im Allgemeinen in dispergierter, d.h. nicht-gelöster Form vor. Bei den erfmdungsgemäßen kosmetischen Zusammensetzungen handelt es sich insbesondere um wasser-enthaltende, d.h. wässrige Zusammensetzungen, in denen das Polyurethan dispergiert vorliegt. Wasser bildet im Allgemeinen neben wahlweise vorhandenen anderen flüssigen Medien, wie beispielsweise Lösungsmitteln, den Hauptbestandteil (> 50 Gew.-%) bezogen auf die flüssigen Dispergiermedien in den erfindungsgemäßen kosmetischen Zusammensetzungen, gegebenenfalls auch das alleinige flüssige Dispergiermedium.

Das erfindungsgemäß verwendete Polyurethan verleiht der kosmetischen Zusammensetzung insbesondere filmbildende Eigenschaften, d.h. beim Auftrag der Zusammensetzung auf die Haut bildet sich ein zusammenhängender Film, der die Eignung der kosmetischen Zusammensetzung als abziehfähige Maskenformulierung begründet, wie weiter unten noch näher beschrieben wird.

Die in den erfindungsgemäßen kosmetischen Zusammensetzungen enthaltenen Polyurethane enthalten mindestens eine Sequenz, die ausgewählt wird aus der Gruppe, die besteht aus: Polyether-, Polycarbonat- und Polyether-Polycarbonat-Sequenzen. Dies bedeutet erfindungsgemäß, dass die Polyurethane Ether- und/oder Carbonat-haltige Wiederholungseinheiten enthalten. Die Polyurethane können ausschließlich Polyether-Sequenzen oder ausschließlich Polycarbonatsequenzen enthalten. Sie können aber auch sowohl Polyether- als auch Polycarbonat-Sequenzen aufweisen, wie sie beispielsweise bei der Herstellung von Polycarbonat-Polyolen unter Verwendung von Polyetherdiolen gebildet werden, wie unten noch ausführlich beschrieben wird. Zusätzlich können sie Polyether-Polycarbonat-Sequenzen aufweisen, welche aus der Verwendung von Polyether-Polycarbonat-Polyolen, wie weiter unten näher beschrieben, hervorgehen.

Besonders bevorzugte Polyurethane werden unter Verwendung von polymeren Polyether-Polyolen und/oder polymeren Polycarbonat-Polyolen und/oder Polyether-Polycarbonat-Polyolen erhalten, welche jeweils zahlenmittlere Molekulargewichte von etwa 400 bis etwa 8000 g/mol (hier und bei den folgenden Molekulargewichtangaben bestimmt durch Gelpermeationschromatographie gegenüber Polystyrol-Standard in Tetrahydofuran bei 23°C) aufweisen. Ihre Verwendung bei der Herstellung der Polyurethane führt durch Umsetzung mit Polyisocyanaten zur Bildung entsprechender Polyether- und/oder Polycarbonat und/oder Polyether-Polycarbonat-Sequenzen in den Polyurethanen mit entsprechendem Molgewicht dieser Sequenzen. Besonders bevorzugt sind erfindungsgemäß Polyurethane, die aus polymeren PolyetherDiolen und/oder polymeren Polycarbonat-Diolen und/oder Polyether-Polycarbonat-Polyolen mit linearem Aufbau erhalten werden.

Die erfindungsgemäßen Polyurethane sind bevorzugt im Wesentlichen lineare Moleküle, können jedoch auch verzweigt sein, was weniger bevorzugt ist.

Das zahlenmittlere Molekulargewicht der erfindungsgemäß bevorzugt verwendeten Polyurethane beträgt beispielsweise etwa von 1000 bis 100000, bevorzugt von 5000 bis 50000.

Die in den erfindungsgemäßen kosmetischen Zusammensetzungen enthaltenen Polyurethane werden den genannten Zusammensetzungen insbesondere als wässrige Dispersionen zugesetzt. Derartige wässrige Polyurethan-Dispersionen sind insbesondere erhältlich durch ein Verfahren, welches die Umsetzung folgender Komponenten umfasst:
A1) ein oder mehrere organische Polyisocyanate,
A2a) ein oder mehrere polymere Polyether-Polyole und/oder ein oder mehrere polymere Polycarbonat-Polyole mit zahlenmittleren Molekulargewichten von bevorzugt jeweils etwa 400 bis 8000 g/mol, bevorzugter etwa 400 bis 6000 g/mol und besonders bevorzugt von etwa 600 bis 3000 g/mol, welche bevorzugt OH-Funktionalitäten von 1,5 bis 6, bevorzugter 1,8 bis 3, besonders bevorzugt von 1,9 bis 2,1 aufweisen,
A2b) gegebenenfalls ein oder mehrere weitere polymere, von A2a) verschiedene Polyole mit zahlenmittleren Molekulargewichten von bevorzugt etwa 400 bis 8000 g/mol, bevorzugter etwa 400 bis 6000 g/mol und besonders bevorzugt von etwa 600 bis 3000 g/mol, welche bevorzugt OH-Funktionalitäten von 1,5 bis 6, bevorzugter 1,8 bis 3, besonders bevorzugt von 1,9 bis 2,1 aufweisen,
A3) gegebenenfalls ein oder mehrere hydroxyfunktionelle Verbindungen, bevorzugt mit Molekulargewichten von 32 bis weniger als 400 g/mol,
A4) gegebenenfalls ein oder mehrere isocyanatreaktive Hydrophilierungsmittel, welche bevorzugt aus anionischen, potentiell anionischen und/oder nichtionischen Hydrophilierungsmitteln ausgewählt werden, und
B1) gegebenenfalls ein oder mehrere aminofunktionelle Verbindungen, bevorzugt mit Molekulargewichten von 32 bis 400 g/mol.

Die in den erfindungsgemäßen, kosmetischen Zusammensetzungen enthaltenen wässrigen Polyurethan-Dispersionen sind bevorzugter erhältlich durch ein Verfahren, welches die folgenden Verfahrensschritte umfasst:
A) Verfahren zur Herstellung eines isocyanatfunktionellen Prepolymers, welches die Umsetzung folgender Komponenten umfasst:
   A1) ein oder mehrere organische Polyisocyanat,
   A2a) ein oder mehrere polymere Polyether-Polyole und/oder ein oder mehrere polymere Polycarbonat-Polyole mit zahlenmittleren Molekulargewichten von bevorzugt jeweils etwa 400 bis 8000 g/mol, bevorzugter etwa 400 bis 6000 g/mol und besonders bevorzugt von etwa 600 bis 3000 g/mol, welche bevorzugt OH-Funktionalitäten von 1,5 bis 6, bevorzugter 1,8 bis 3, besonders bevorzugt von 1,9 bis 2,1 aufweisen,
   A2b) gegebenenfalls ein oder mehrere weitere polymere, von A2a) verschiedene Polyole mit zahlenmittleren Molekulargewichten von bevorzugt etwa 400 bis 8000 g/mol, bevorzugter etwa 400 bis 6000 g/mol und besonders bevorzugt von etwa 600 bis 3000 g/mol, welche bevorzugt OH-Funktionalitäten von 1,5 bis 6, bevorzugter 1,8 bis 3, besonders bevorzugt von 1,9 bis 2,1 aufweisen,
   A3) gegebenenfalls ein oder mehrere hydroxyfunktionelle Verbindungen, bevorzugt mit Molekulargewichten von 32 bis weniger als 400 g/mol,
   A4) gegebenenfalls ein oder mehrere isocyanatreaktive Hydrophilierungsmittel, welche bevorzugt aus anionischen, potentiell anionischen und/oder nichtionischen Hydrophilierungsmitteln ausgewählt werden,
   (wobei die Stöchiometrie so gewählt wird, dass die entstehenden Prepolymere isocyanatterminiert sind), und
B) Verfahren zur Herstellung des Polyurethans, welches umfasst:
   Die Umsetzung des in Schritt A) erhaltenen isocyanatfunktionellen Prepolymers mit einer oder mehreren der Komponenten, welche aus der Gruppe ausgewählt werden, die besteht aus:
      B1) ein oder mehreren aminofunktionellen Verbindungen, bevorzugt mit Molekulargewichten von 32 bis 400 g/mol und
      B2) ein oder mehreren isocyanatreaktiven, bevorzugt aminofunktionellen Hydrophilierungsmitteln, welche bevorzugt aus anionischen und potentiell anionischen Hydrophilierungsmitteln ausgewählt werden.

In Schritt B) erfolgt im Allgemeinen eine Kettenverlängerung des in Schritt A) erhaltenen isocyanatfunktionellen Prepolymers.

Insbesondere umfasst die aminofunktionelle Verbindung B) wenigstens eine aminofunktionelle Verbindung B1), die keine ionischen und/oder ionogene Gruppen aufweist. Hierbei handelt es sich bevorzugt um ein Diamin, welches keine ionischen und/oder ionogenen Gruppen aufweist. Bevorzugt werden in Schritt B) sowohl die Komponente B1) als auch die Komponente B2) verwendet.

Die Dispergierung der Polyurethane in Wasser kann vor, während oder nach Schritt B) erfolgen, wobei gegebenenfalls enthaltene, potentiell ionische Gruppen durch teilweise oder vollständige Umsetzung mit einem Neutralisationsmittel in die ionische Form überführt werden.

Um die erfindungsgemäß bevorzugte anionische Hydrophilierung zu erreichen, müssen die anionischen oder potentiell anionischen Komponenten A4) und/oder B2) als Hydrophilierungsmittel eingesetzt werden, was bevorzugt der Fall ist. Diese weisen bevorzugt wenigstens eine gegenüber NCO-Gruppen reaktive Gruppe wie bevorzugt eine Amino-, Hydroxy- oder Thiolgruppen auf und darüber hinaus wenigstens eine anionische Gruppe oder in eine anionische Gruppe überführbar Gruppe auf, wie -COO⁻, -SO₃⁻ oder -PO₃²⁻ bzw. deren ganz oder teilweise protonierte Säureformen. Dies wird weiter unten noch näher ausgeführt. Besonders bevorzugt sind Sulfonatgruppen als anionische Gruppen. Die Verwendung von Sulfonatgruppen als anionische Gruppen in den erfindungsgemäß verwendeten Polyurethanen führt zu einer größeren Beständigkeit gegenüber Elektrolyten, wie Salzen, die Einstellung eines neutralen pH-Wertes der Dispersionen wird vereinfacht, und die Dispersionenen bleiben über einen breiteren pH-Wert wie einem pH von etwa 5 bis 8 stabil. Auch wird durch die Verwendung der Sulfonatgruppen-tragenden Polyurethane die Stabilität der erfindungsgemäßen kosmetischen Zusammensetzungen erhöht.

Die bevorzugten wässrigen, anionischen Polyurethan-Dispersionen haben einen niedrigen Grad an hydrophilen anionischen Gruppen, bevorzugt von 0,1 bis 15 Milliequivalenten pro 100 g Festharz. Ein höherer Grad an anionischen Gruppen ist nachteilig weil in einigen Fällen die Trockung des resultierenden Films aus der kosmetischen Zusammensetzung verlangsamt werden könnte.

Um eine gute Sedimentationsstabilität zu erreichen, liegt die zahlenmittlere Teilchengröße der speziellen Polyurethan-Dispersionen bevorzugt bei weniger als 750 nm, besonders bevorzugt bei weniger als 500 nm und ganz besonders bevorzugt weniger als 400 nm, bestimmt mittels Laserkorrelations-Spektroskopie nach Verdünnung mit entionisiertem Wasser (Gerät: Malvern Zetasizer 1000, Malver Inst. Limited).

Im Schritt A) zur Herstellung der erfindungsgemäß verwendeten Polyurethane beträgt das molare Verhältnis von NCO-Gruppen der Verbindungen aus Komponente A1) zu NCO-reaktiven Gruppen wie Amino-, Hydroxy- oder Thiolgruppen der Verbindungen der Komponenten A2) bis A4) bei der Herstellung des NCO-funktionellen Prepolymers bevorzugt 1,05 bis 3,5, bevorzugter 1,2 bis 3,0 besonders bevorzugt 1,3 bis 2,5.

Die aminofunktionellen Verbindungen in Stufe B) werden in solch einer Menge eingesetzt, dass das Äquivalentverhältnis von isocyanatreaktiven Aminogruppen dieser Verbindungen zu den freien Isocyanatgruppen des Prepolymers bevorzugt 40 bis 150 %, bevorzugter zwischen 50 bis 125 %, besonders bevorzugt zwischen 60 bis 120 % beträgt.

Geeignete Polyisocyanate der Komponente A1) sind insbesondere die dem Fachmann an sich bekannten aromatischen, araliphatischen, aliphatischen oder cycloaliphatischen Polyisocyanate einer NCO-Funktionalität von insbesondere 2.

Beispiele solcher geeigneten Polyisocyanate sind 1,4-Butylendiisocyanat, 1,6-Hexamethylendiisocyanat (HDI), Isophorondiisocyanat (IPDI), 2,2,4 und/oder 2,4,4-Trimethylhexamethylendiisocyanat, die isomeren Bis(4,4'-isocyanatocyclohexyl)methane oder deren Mischungen beliebigen Isomerengehalts, 1,4-Cylohexylendiisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis(isocyanatomethyl)benzol (XDI), sowie Alkyl-2,6-diisocyanatohexanoate (Lysindiisocyanate) mit C1-C8-Alkylgruppen.

Neben den vorstehend genannten Polyisocyanaten können anteilig auch modifizierte Diisocyanate mit Uretdion-, Isocyanurat-, Urethan-, Allophanat-, Biuret-, Iminooxadiazindion- und/oder Oxadiazintrionstruktur sowie nicht-modifiziertes Polyisocyanat mit mehr als 2 NCO-Gruppen pro Molekül sei z.B. 4-Isocyanalomethyl-1,8-octandiisocyanat (Nonantriisocyanat) oder Triphenylmethan-4,4',4"-triisocyanat mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von 2 bis 4, bevorzugter 2 bis 2,6 und besonders bevorzugt 2 bis 2,4, ganz besonders bevorzugt 2. Eine Funktionalität von ungefähr 2 ist bevorzugt, weil dadurch geeignete mechanische Eigenschaften resultieren.

Besonders bevorzugt werden in A1) 1,6-Hexamethylendiisocyanat, Isophorondiisocyanat, die isomeren Bis(4,4'-isocyanatocyclohexyl)methane, sowie deren Mischungen eingesetzt.

Als Komponente A2a) werden bevorzugt polymere Polyether-Polyolen und/oder Polycarbonat-Polyole jeweils mit einem zahlenmittleren Molekulargewicht Mₙ von etwa 400 bis 8000 g/mol, bevorzugter von etwa 400 bis 6000 g/mol und besonders bevorzugt von 600 bis 3000 g/mol eingesetzt. Der Ausdruck "polymere" bedeutet hier insbesondere, dass die genannten Polyether-Polyole und/oder Polycarbonat-Polyol mindestens zwei miteinander verbundene Ethergruppen bzw. Carbonatgruppen enthaltende Wiederholungseinheiten aufweisen. Der Ausdruck "und/oder" bedeutet im Rahmen der vorliegenden Erfindung, dass es sich bei Komponente A2a) um Polyether-Polyole, Polycarbonat-Polyole, Polyether-Polycarbonat-Polyole und deren Mischungen handeln kann. Letztere Polyole weisen sowohl Ethergruppen- als auch Carbonatgruppen-enthaltende Wiederholungs-Sequenzen auf. Erfindungsgemäß sind sämtliche dieser Möglichkeiten zur Bildung der erfindungsgemäße verwendeten Polyurethane mit wenigstens einer Sequenz, die ausgewählt wird aus der Gruppe, die besteht aus: Polyether- und Polycarbonat-Sequenzen eingeschlossen.

Die Bestimmung des zahlenmittleren Molekulargewicht Mₙ der Polyether-Polyole, der Polycarbonat-Polyole sowie der Polyether-Polycarbonat-Polyole erfolgt dabei wie oben angeben durch Gelpermeationschromatographie gegenüber Polystyrol-Standard in Tetrahydofuran bei 23°C.

Diese weisen bevorzugt eine OH-Funktionalität von 1,5 bis 6, bevorzugter von 1,8 bis 3, besonders bevorzugt von 1,9 bis 2,1, ganz besonders bevorzugt von 2 auf. Die Verbindungen der Komponente A2a) können einzeln oder in beliebigen Mischungen untereinander eingesetzt werden. In bevorzugten Ausführungsformen der Erfindung handelt es sich bei Komponente A2a) um:
- Mischungen, enthaltend wenigstens ein Polyether-Polyol und wenigstens ein Polycarbonat-Polyol,
- besonders bevorzugt Mischungen, enthaltend mehr als ein Polyether-Polyol, bzw. ein Gemisch mehrerer Polyether-Polyole mit unterschiedlichen Molekulargewichten, wobei es sich insbesondere um Poly(tetramethylenglykol)polyetherpolyole (wie (HO-(CH₂-CH₂-CH₂-CH₂-O)ₓ-H) handelt, sowie
- Mischungen, enthaltend mehr als ein Polyether-Polyol, und wenigstens ein Polycarbonat-Polyol.

Es können insbesondere Hydroxylgruppen aufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind insbesondere durch Reaktion von Kohlensäurederivaten, wie Diphenylcarbonat, Dimethylcarbonat oder Phosgen, mit Polyolen, bevorzugt Diolen, erhältlich. Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält das Polycarbonat-Polyol, insbesondere das Polycarbonatdiol 40 bis 100 Gew.-% Hexandiol, bevorzugt 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden. Derartige Polyether-Polycarbonatdiole weisen sowohl Ethergruppenals auch Carbonatgruppen-haltige Wiederholungseinheiten auf. Sie werden beispielweise erhalten durch Umsetzung von Polyetherpolyolen mit Carbonat-Vorläufern, wie Phosgen etc.

Die Hydroxylgruppen aufweisenden Polycarbonate sind bevorzugt linear gebaut. Bevorzugt ist ihre OH-Funktionalität etwa 2.

Ebenfalls können als Komponente A2a) Polyetherpolyole eingesetzt werden.

Geeignet sind insbesondere die in der Polyurethanchemie an sich bekannten Polytetramethylenglykolpolyether wie sie beispielsweise durch Polymerisation von Tetrahydrofuran mittels kationischer Ringöffnung erhältlich sind, insbesondere mit den oben genannten bevorzugten Molekulargewichten.

Ebenfalls geeignete Polyetherpolyole sind die an sich bekannten Additionsprodukte von Styroloxid, Ethylenoxid, Propylenoxid, Butylenoxid und/oder Epichlorhydrin an di- oder polyfunktionelle Startermoleküle. So sind insbesondere Polyalkylenglykole, wie Polyethylen-, Polypropylen- und/oder Polybutylenglykole anwendbar, insbesondere mit den oben genannten bevorzugten Molekulargewichten.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol und Butyldiglykol.

In einer besonders bevorzugten Ausführungsformen enthalten die erfindungsgemäß verwendeten Polyurethane als Komponente A2a) eine Mischung aus Polycarbonatpolyolen und Polytetramethylenglykolpolyolen, wobei in dieser Mischung bevorzugt der Anteil an Polycarbonat-polyolen in der Mischung von mehr als 0 bis 80 Gew.-% und der Anteil an Polytetramethylenglykolpolyolen von weniger als 100 bis 20 Gew.-%, bezogen auf die Komponente A2a), beträgt. In einer weiteren besonders bevorzugten Ausführungsform der Erfindung enthalten die Polyurethane als Komponente A2a) ausschließlich Polytetramethylenglykolpolyole, besonders bevorzugt Mischungen der genannten Polytetramethylenglykolpolyole bzw. diole mit unterschiedlichen zahlenmittleren Molekulargewichten. Die Verwendung der daraus resultierenden Poly(tetramethylenglykol)polyether-Polyurethane ist erfindungsgemäß besonders bevorzugt, da sie zur Bildung von Filmen mit besonders hoher Reißdehnung Führt.

Als wahlweise zur Herstellung der Polyurethane eingesetzte Komponente A2b) werden von der Komponente A2a) verschiedene polymere Polyole, bevorzugt mit einem zahlenmittleren Molekulargewicht Mₙ von bevorzugt etwa 400 bis 8000 g/mol, bevorzugter etwa von 400 bis 6000 g/mol und besonders bevorzugt von etwa 600 bis 3000 g/mol eingesetzt. Das zahlenmittlere Molekulargewicht wird dabei bestimmt wie bei der Beschreibung der Komponente A2a) angegeben. Der Ausdruck "polymere" bedeutet dabei, dass es sich um Polyole handelt, die wenigstens zwei miteinander verbundene Wiederholungseinheiten aufweisen.

Bevorzugt wird die Komponente A2b) nicht zur Herstellung der erfindungsgemäß verwendeten Polyurethane verwendet, entsprechend zweckmäßig weniger als 1 Gew.-% bezogen auf das Polyurethan.

Diese als Komponente A2b) verwendeten Verbindungen weisen bevorzugt eine OH-Funktionalität von 1,5 bis 6, besonders bevorzugt von 1,8 bis 3, ganz besonders bevorzugt von 1,9 bis 2,1 auf.

Solche polymeren Polyole sind insbesondere die in der Polyurethanlacktechnologie an sich bekannten Polyesterpolyole, Polyacrylatpolyole, Polyurethanpolyole, Polyesterpolyacrylatpolyole, Polyurethanpolyacrylatpolyole und Polyurethanpolyesterpolyole Diese können einzeln oder in beliebigen Mischungen untereinander eingesetzt werden.

Die genannten Polyesterpolyole sind beispielsweise die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole, welche für die Herstellung der Komponente A2b) geeignet sind, schließen ein: Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopenthylglykolester bevorzugt sind. Daneben können auch Polyole wie Trimethylolpropan, Glycerin, Erythrit, Pentaerythrit, Triemthylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols größer als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Die gegebenenfalls als Komponente A3) verwendeten hydroxyfunktionellen Verbindungen besitzen zweckmäßig Molekulargewichte von 62 bis weniger als 400 g/mol. Im Unterschied zu den Komponenten A2) (A2a) und A2b)) handelt es sich bei der Komponente A3) insbesondere nicht um polymere hydrofunktionelle Verbindungen sondern um monomere Verbindungen, welche keine Wiederholungseinheiten wie die Verbindungen der Komponente A2) aufweisen. Als Komponente A3) können insbesondere Polyole, bevorzugt des genannten Molekulargewichtsbereichs, bevorzugt mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit sowie deren beliebige Mischungen untereinander eingesetzt werden.

Geeignet sind als Komponente A3) auch Esterdiole des genannten Molekulargewichtsbereichs wie α-Hydroxybutyl-ε-hydroxycapronsäureester, ω-Hydroxyhexyl-y-hydroxybuttersäure-ester, Adipinsäure-(β-hydroxyethyl)ester oder Terephthalsäurebis(β-hydroxyethyl)-ester.

Ferner können als Komponente A3) auch monofunktionelle, isocyanatreaktive, Hydroxylgruppen-haltige Verbindungen eingesetzt werden. Beispiele solcher monofunktionellen Verbindungen sind Ethanol, n-Butanol, Ethylenglykolmonobutylether, Diethylenglykolmonomethylether, Ethylenglykolmonobufiylether, Diethylenglykolmonobutylether, Propylenglykolmonomethylether, Dipropylenglykol-monomethylether, Tripropylenglykolmonomethylether, Dipropylenglykohnonopropylether, Propylenglykolmonobutylether, Dipropylenglykolmonobutylether, Tripropylenglylcohnonobutylether, 2-Ethylhexanol, 1-Octanol, 1-Dodecanol, 1-Hexadecanol.

Bevorzugte Verbindungen der Komponente A3) sind 1,6-Hexandiol, 1,4-Butandiol, Neopentylglykol und Trimethylolpropan.

In einer bevorzugten Ausführungsform der Erfindung enthält dass erfindungsgemäß verwendete Polyurethan weniger als etwa 10 Gew.-% der Komponente A3), bevorzugt weniger als 5 Gew.-% der Komponente A3), jeweils bezogen auf die Gesamtmasse des Polyurethans, noch bevorzugter wird Komponente A3) zur Herstellung des Polyurethan nicht verwendet.

Als Komponente A4) werden zur Herstellung der erfindungsgemäß verwendeten Polyurethane gegebenenfalls ein oder mehrere isocyanatreaktive Hydrophilierungsmittel verwendet, welche bevorzugt aus anionischen, potentiell anionischen und/oder nichtionischen Hydrophilierungsmitteln ausgewählt werden. Die als Komponente A4) verwendeten isocyanatreaktiven Hydrophilierungsmittel sind insbesondere von den Komponenten A2) (einschließlich A2a) und A2b)) und A3)) verschieden.

Unter anionisch bzw. potentiell anionisch hydrophilierenden Verbindungen der Komponente A4) werden insbesondere sämtliche Verbindungen verstanden, die mindestens eine isocyanatreaktive Gruppe wie eine Hydroxylgruppe oder eine Aminogruppe aufweisen sowie mindestens eine Funktionalität wie z.B. -COO⁻M⁺, -SO₃⁻M⁺, -PO(O⁻M⁺)₂ mit M⁺ beispielsweise gleich Metallkation, insbesondere Alkalimetallkation, wie Na⁺, H⁺, NH₄⁺, NHR₃⁺, wobei R jeweils ein C1-C12-Alkylrest, C5-C6-Cycloalkylrest und/oder ein C2-C4-Hydroxyalkylrest sein kann. Diese Verbindungen gehen, wie dem Fachmann bekannt ist, bei Wechselwirkung mit wässrigen Medien ein pH-Wert-abhängiges Dissoziationsgleichgewicht ein und können auf diese Weise negativ oder neutral geladen sein. Geeignete an ionisch oder potentiell anionisch hydrophilierende Verbindungen schließen insbesondere ein: Mono- und Dihydroxycarbonsäuren, Mono- und Dihydroxysulfonsäuren, sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und NaHSO₃, wie es in DE-A 2 446 440, Seite 5 - 9, Formel I-III beschrieben ist. Bevorzugte anionische oder potentiell anionische Hydrophilierungsmittel der Komponente A4) sind solche der vorstehend genannten Art, die über Carboxylat- bzw Carbonsäuregruppen und/oder Sulfonatgruppen verfügen.

Besonders bevorzugte anionische oder potentiell anionische Hydrophilierungsmittel A4) sind solche, die Carboxylat- bzw Carbonsäuregruppen als ionische oder potentiell ionische Gruppen enthalten, wie Dimethylolpropionsäure, Dimethylolbuttersäue und Hydroxypivalinsäure bzw. deren Salze.

Geeignete nichtionisch hydrophilierende Verbindungen der Komponente A4) schließen insbesondere auch Polyoxyalkylenether ein, welche über isocyanat-reaktive Gruppen, wie Hydroxy-und/oder Aminogruppen verfügen. Bevorzugt handelt es sich um einen monofunktionellen Polyoxyalkylenether, insbesondere um einen monohydroxyfunktionellen Polyoxyalkylenether, wie beispielsweise ein monohydroxyfunktioneller Polyether auf Ethylen-/Propylenoxid-Basis, wie der von der Anmelderin vertriebene Polyether LB 25.

Beispiele der nichtionisch hydrophilierenden Verbindungen als Komponente A4) sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle, insbesondere auch monofunktioneller Alkohole, zugänglich sind (z.B. in Ullmanns Encyclopädie der technischen Chemie, 4. Auflage, Band 19, Verlag Chemie, Weinheim S. 31-38).

Diese sind insbesondere entweder reine Polyethylenoxidether oder gemischte Polyalkylenoxidether, insbesondere Polyethylenoxid-/Polypropylenoxid-Polyether-Verbindungen, die blockartig oder statistisch aufgebaut sein können, wobei sie bevorzugt mindestens 30 mol-%, bevorzugt mindestens 40 mol-% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis weniger als 100 mol-% Ethylenoxid- und mehr als 0 bis 60 mol% Propylenoxideinheiten aufweisen.

Geeignete Startermoleküle für solche nichtionischen Hydrophilierungsmittel A4) sind insbesondere gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykolmonoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol. Weniger bevorzugt sind sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Wahlweise kann in Schritt B) die Komponente B1) verwendet werden. Die Komponente B1) wird ausgewählt aus aminofunktionelle Verbindungen, bevorzugt mit Molekulargewichten von 32 bis 400 g/mol. Die Komponente B1) ist von der Komponente A4) und der Komponenten B2) verschieden. Sie unterscheidet sich von der Komponente A4) und der Komponenten B2) bevorzugt dadurch, dass sie keine anionischen und potentiell anionischen Gruppen aufweist.

Als Komponente B1) können insbesondere Di- oder Polyamine wie 1,2-Ethylendiamin, 1,2-und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4-Diaminodicyclohexylmethan und/oder Dimethylethylendiamin eingesetzt werden. Ebenfalls möglich ist die Verwendung von Hydrazin oder sowie Hydraziden wie Adipinsäuredihydrazid. Bevorzugt sind Isophorondiamin, 1,2-Ethylendiamin, 1,4-Diaminobutan, Hydrazin und Diethylentriamin.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethyl-aminopropan, 3-Amino-1-cyclohexylaminopropan, 3-Amino-1-Methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin.

Bevorzugte Verbindungen der Komponente B1) sind 1,2-Ethylendiamin, 1,4-Diaminobutan und Isophorondiamin.

Bevorzugt kann in Schritt B) die Komponente B2) verwendet werden. Die Komponente B2) wird ausgewählt unter isocyanatreaktiven, bevorzugt aminofunktionellen Hydrophilierungsmitteln, welche bevorzugt aus ionischen und/oder ionogenen, insbesondere anionischen und potentiell anionischen Hydrophilierungsmitteln ausgewählt werden. Dabei kann es sich um die gleichen Verbindungen handeln, die wahlweise in Schritt A) als Komponente A4) verwendet werden können, mit Ausnahme der nicht-ionischen Hydrophilierungsmittel. Als Komponente A4) und Komponente B2) können daher in einem Verfahren zur Herstellung des erfindungsgemäß verwendeten Polyurethans gleiche oder verschiedene Verbindungen verwendet werden.

Hinsichtlich der anionisch bzw. potentiell anionisch hydrophilierenden Verbindungen der Komponente B2) kann daher an sich zu den für Komponente A4) gegebenen Definitionen verwiesen werden. Die Komponente B2) schließt insbesondere sämtliche Verbindungen ein, die mindestens eine isocyanatreaktive Gruppe, bevorzugt eine Amino-Gruppe aufweisen, sowie mindestens eine anionische Funktionalität wie z.B. -COO⁻M⁺, -SO₃⁻M⁺, -PO(O⁻M⁺)₂ mit M⁺ beispielsweise gleich Metallkation, wie Alkalimetall-Kation, wie Na⁺, H⁺, NH₄⁺, NHR₃⁺, wobei R jeweils ein C1-C12-Alkylrest, C5-C6-Cycloalkylrest und/oder ein C2-C4-Hydroxyalkylrest sein kann. Wie dem Fachmann bekannt ist, gehen diese Gruppen bei der Wechselwirkung mit wässrigen Medien ein pH-Wert-abhängiges Dissoziationsgleichgewicht ein, und können auf diese Weise negativ oder neutral geladen sein.

Besonders als Komponente B2) geeignete anionisch oder potentiell anionisch hydrophilierende Verbindungen sind Mono- und Diaminocarbonsäuren, Mono- und Diaminosulfonsäuren sowie Mono- und Diaminophosphonsäuren und ihre Salze. Beispiele solcher anionischen bzw. potentiell anionischen Hydrophilierungsmittel sind N-(2-Aminoethyl)-β-alanin, 2-(2-Amino-ethylamino)-ethansulfonsäure, Ethylendiamin-propyl- oder -butylsulfonsäure, 1,2- oder 1,3-Propylendiamin-β-ethylsulfonsäure, Glycin, Alanin, Taurin, Lysin, 3,5-Diaminobenzoesäure und das Additionsprodukt von IPDA und Acrylsäure (EP-A 0 916 647, Beispiel 1). Weiterhin kann das aus aus WO-A 01/88006 bekannte Cylohexylaminopropansulfonsäure (CAPS) als anionisches oder potentiell anionisches Hydrophilierungsmittel verwendet werden.

Bevorzugte anionische oder potentiell anionische Hydrophilierungsmittel der Komponente B2) sind solche der vorstehend genannten Art, die über Carboxylat- bzw. Carbonsäuregruppen und/oder Sulfonatgruppen verfügen wie die Salze von N-(2-Aminoethyl)-β-alanin, der 2-(2-Aminoethylamino)ethansulfonsäure oder des Additionsproduktes von IPDA und Acrylsäure (EP-A 0 916 647, Beispiel 1).

Zur Hydrophilierung können auch Mischungen aus anionischen bzw. potentiell anionischen Hydrophilierungsmitteln und nichtionischen Hydrophilierungsmitteln verwendet werden.

Das erfindungsgemäß verwendete Polyurethan enthält bevorzugt ein anionisches bzw. potentiell anionisches Hydrophilierungsmittel als Komponente A4) (dann wird es bereits bei der Prepolymerbildung umgesetzt) oder als Komponente B2) (dann wird es erst bei der Umsetzung des Prepolymers eingesetzt). Bevorzugt wird bei der Umsetzung des Prepolymers in Schritt B) ein anionisches bzw. potentiell anionisches Hydrophilierungsmittel als Komponente B2) zugesetzt. Mit anderen Worten enthält das erfindungsgemäß verwendete Polyurethan in einer bevorzugten Ausführungsform eine anionische oder potentiell anionische, d.h., in ein Polyurethan-Anion dissoziierbare Gruppe. Die besonders bevorzugte anionische bzw. potentiell anionische Gruppe ist die Sulfonat-Gruppe: -SO₃⁻M⁺. Diese wird besonders bevorzugt mittels eines Diaminosulfonats, insbesondere mittels des NH₂-CH₂CH₂-NH-CH₂CH₂-SO₃Na in das Polyurethan eingebracht.

In einer bevorzugten Ausführungsform werden zur Herstellung der speziellen Polyurethan-Dispersionen sowohl die Komponente B1) als auch die Komponente B2) verwendet. Durch die Verwendung der Komponente B1) kann eine hohe Molmasse aufgebaut werden, ohne dass die Viskosität des zuvor hergestellten isocyanatfunktionellen Präpolymers in einem Maße ansteigt, welches einer Verarbeitung entgegenstehen würde. Durch die Verwendung der Kombination der Komponenten B1) und B2) läßt sich insbesondere eine optimale Balance zwischen Hydrophilie und Kettenlänge einstellen.

In einer bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% addieren:
5 bis 40 Gew.-% Komponente A1),
55 bis 90 Gew.% A2a),
0 bis 50 Gew.-% A2b),
0,5 bis 20 Gew.-% Summe der Komponenten A3) und/oder B1)
0,1 bis 25 Gew.-% Summe der Komponenten Komponente A4) und/oder B2), wobei bezogen auf die Gesamtmengen der Komponenten A1) bis A4) und B1) bis B2) besonders bevorzugt 0,1 bis 5 Gew.-% an anionischen bzw. potentiell anionischen Hydrophilierungsmitteln aus A4) und/oder B2) verwendet werden.

In einer besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
5 bis 35 Gew.-% Komponente A1),
60 bis 90 Gew.-% A2a),
0 bis 30 Gew.-% A2b),
0,5 bis 15 Gew.-% Summe der Komponenten A3) und/oder B1)
0,1 bis 15 Gew.-% Summe der Komponenten Komponente A4) und/oder B2), wobei bezogen auf die Gesamtmengen der Komponenten A1) bis A4) und B1) bis B2) 0,2 bis 4 Gew.-% an anionischen bzw. potentiell anionischen Hydrophilierungsmitteln aus A4) und/oder B2) verwendet werden.

In einer ganz besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
10 bis 30 Gew.-% Komponente A1),
65 bis 85 Gew.-% A2a),
0 Gew.-% A2b),
0,5 bis 14 Gew.-% Summe der Komponenten A3) und/oder B1),
0,1 bis 13,5 Gew.-% Summe der Komponenten A4) und/oder B2), wobei bezogen auf die Gesamtmengen der Komponenten A1) bis A4) und B1) bis B2) 0,5 bis 3,0 Gew.-% an anionischen bzw. potentiell anionischen Hydrophilierungsmitteln aus A4) und/oder B2) verwendet werden.

Die Herstellung der bevorzugt anionisch hydrophilierten Polyurethan-Dispersionen kann bevorzugt in einer oder mehreren Stufe(n) in homogener oder bei mehrstufiger Umsetzung, teilweise in disperser Phase durchgeführt werden. Nach vollständig oder teilweise durchgeführter Polyaddition in Schritt A) (Komponenten A1) bis A4)) erfolgt bevorzugt ein Dispergier-, Emulgier-oder Lösungsschritt. Im Anschluss erfolgt gegebenenfalls eine weitere Polyaddition oder Modifikation in disperser Phase.

Dabei können alle aus dem Stand der Technik bekannten Verfahren wie z. B. Prepolymer-Mischverfahren, Acetonverfahren oder Schmelzdispergierverfahren verwendet werden. Bevorzugt wird das Aceton-Verfahren verwendet.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon.

Andere Lösemittel wie Xylol, Toluol, Cyclohexan, Butylacetat, Methoxypropylacetat, N-Methylpyrrolidon, N-Ethylpyrrolidon, Lösemittel mit Ether- oder Estereinheiten können zusätzlich eingesetzt und ganz oder teilweise abdestilliert werden oder vollständig im Falle von, N-Methylpyrrolidon, N-Ethylpyrrolidon in der Dispersion verbleiben. Bevorzugt werden aber außer den üblichen aliphatischen, ketofunktionellen Lösemitteln keine anderen Lösungsmittel verwendet. Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanatgruppen zu isocyanatreaktiven Gruppen bevorzugt 1,05 bis 3,5, bevorzugter 1,2 bis 3,0, besonders bevorzugt 1,3 bis 2,5. Bevorzugt werden also Isocyanatfunktionelle Prepolymere gebildet, welche anschließend bevorzugt mit aminofunktionellen Verbindungen B1) und/oder B2) umgesetzt werden.

Die Umsetzung der Komponenten A1) bis A4) zum Prepolymer erfolgt teilweise oder vollständig, bevorzugt aber vollständig. Es werden so bevorzugt Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-methylmorpholin, Methyldiisopropylamin, Ethyldiisopropylamin und Diisopropylethylamin. Die Alkylreste können beispielsweise auch Hydroxylgruppen tragen, wie bei den Dialkylmonoalkanol-, Alkyldialkanol- und Trialkanolaminen. Als Neutralisationsmittel sind gegebenenfalls auch anorganische Basen, wie wässrige Ammoniaklösung oder Natrium- bzw. Kaliumhydroxid einsetzbar.

Bevorzugt sind Ammoniak, Triethylamin, Triethanolamin, Dimethylethanolamin oder Diisopropylethylamin sowie Natriumhydroxid und Kaliumhydroxid, besonders bevorzugt sind Natriumhydroxid und Kaliumhydroxid.

Die Stoffmenge der Basen beträgt 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, in dem das Dispergierwasser bereits das Neutralisationsmittel enthält.

Im Anschluss wird in einem weiteren Verfahrensschritt, falls noch nicht oder nur teilweise geschehen, das erhaltene Prepolymer bevorzugt mit Hilfe von aliphatischen Ketonen wie Aceton oder 2-Butanon gelöst.

Bei der Kettenverlängerung/-terminierung in Stufe B) werden NH₂- und/oder NH-funktionelle Komponenten mit den noch verbliebenen Isocyanatgruppen des Prepolymers teilweise oder vollständig umgesetzt. Bevorzugt wird die Kettenverlängerung/-terminierung vor der Dispergierung in Wasser durchgeführt.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin verwendet.

Werden zur teilweisen oder vollständigen Kettenverlängerung anionische oder potentiell anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Die aminischen Komponenten B1) und B2) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 70 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene flüchtige Lösemittel wird üblicherweise anschließend destillativ entfernt. Eine Entfernung bereits während der Dispergierung ist ebenfalls möglich.

Nach Abreaktion der Isocyanatgruppen oder nach Dispergierung können auch alkoholfunktionelle Lösemittel zugesetzt werden, beispielsweise als Koaleszenzmittel. Beispiele sind Ethanol, n-Butanol, n-Propanol, Ethylenglykolmonobutylether (2-Butoxy-ethanol), Diethylenglykolmonomethylether, Propylenglykolmonomethylether, Propylenglykolmonobutylether, Dipropylenglykolmonobutylether und Tripropylenglykolmonobutylether.

Die erfindungsgemäß bevorzugt verwendeten Polyurethan-Dispersionen sind im Allgemeinen wässrige Dispersionen. Der pH-Wert der erfindungsgemäß verwendeten Polyurethan-Dispersionen beträgt typischerweise weniger als 9,0, bevorzugt weniger als 8,5, besonders bevorzugt weniger als 8,0 und liegt ganz besonders bevorzugt bei 6,0 bis 7,5.

Der Feststoffgehalt der Polyurethan-Dispersionen, welche zur Herstellung der kosmetischen Zusammensetzung der Erfindung bevorzugt verwendet wird, beträgt im allgemeinen 30 bis 70, bevorzugt 40 bis 65, besonders bevorzugt 45 bis 60 Gew.-%. Die Feststoffgehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.

Der Gehalt an der wässrigen Polyurethan-Dispersionen an der kosmetischen Zusammensetzung zum Auftrag auf die Haut insbesondere auch zur Herstellung der Reinigungsmasken beträgt im allgemeinen 5 bis 90, bevorzugt 25 bis 80, besonders bevorzugt 40 bis 70 Gew.-%.

Die erfindungsgemäßen kosmetischen Zusammensetzungen enthalten bevorzugt 1 bis 100 Gew.-%, besonders bevorzugt 10 bis 90 Gew.-% des Polyurethans, bezogen auf den Festkörpergehalt der kosmetischen Zusammensetzung.

Die kosmetische Zusammensetzung der Erfindung ist im Allgemeinen eine wässrige Zusammensetzung, welche gegebenenfalls auch ein kosmetisch geeignetes Lösungsmittel und/oder Koaleszenzmittel enthalten kann. Das verwendete Wasser in der erfmdungsgemäßen kosmetischen Zusammensetzung kann beispielsweise ein reines demineralisiertes Wasser, Mineralwasser, Thermalwasser, Blütenwasser, Leitungswasser und/oder Meerwasser sein.

Der Wasseranteil der kosmetischen Zusammensetzung kann im Bereich von 40 bis 95 Gew.-%, bevorzugt im Bereich von 50 bis 90 Gew.-%, ganz bevorzugt im Bereich von 60 bis 80 Gew.-% bezogen auf das Gesamtgewicht der kosmetischen Zusammensetzung, liegen.

Die bevorzugten Lösungsmittel sind beispielsweise die aliphatischen Alkohole mit C1-4 Kohlenstoffatomen wie Ethanol, Isopropanol, Polyole, wie Glykole und deren Derivate wie Dipropylenglykol, Butylen-1,3-glykol und deren Gemischen ausgewählt.

Der Mengenanteil des Lösungsmittels oder der Lösungsmittel in der erfindungsgemäßen kosmetischen Zusammensetzung, insbesondere der Reinigungsmaskenzusammensetzung kann beispielsweise im Bereich von 0 bis 20 Gew.-%, bezogen auf das Gesamtgewicht der Zusammensetzung, liegen. Bevorzugt sind keine Lösungsmittel enthalten.

Die erfindungsgemäßen kosmetischen Zusammensetzungen, insbesondere die Reinigungsmaskenzusammensetzungen können vorteilhaft Verdicker enthalten. Vorteilhafte Verdicker sind:
- Vernetzte oder nichtvernetzte Acrylsäure oder Methacrylsäure Homo- oder Copolymere. Hierzu gehören vernetzte Hompolymere von Methacrylsäure oder Acrylsäure, Copolymerisate aus Acrylsäure und/oder Methacrylsäure und Monomeren, die von anderen Acryl- oder Vinylmonomeren abgeleitet sind, wie C10-30 Alkylacrylate, C10-30-Alkylmethacrylate, Vinylacetat und Vinylpyrrolidon.
- Verdickende Polymere natürlicher Herkunft beispielweise auf Cellulosebasis, Guargummi, Xanthan, Scleroglucan, Gellangummi, Rhamsan und Karayagummi, Alginate, Maltodextrin, Stärke und ihre Derivate, Johannisbrotkernmehl, Hyaluronsäure, Carrageenan.
- Nichtionische, anionische, kationische oder amphotere assoziative Polymere z.B. auf Basis von Polyethylenglycole und ihre Derivate, oder Polyurethane.
- Vernetzte oder nichtvernetzte Homopolymere oder Copolymere auf Basis von Acrylamid oder Methacrylamid, wie Homopolymere von 2-Acrylamido-2-methylpropansulfonsäure, Copolymere von Acrylamid oder Methacrylamid und Methacryloyloxyethyltrimethylammoniumchlorid oder Copolymere von Acrylamid und 2-Acrylamido-2-methylpropansulfonsäure.

Besondere vorteilhafte Verdicker sind verdickende Polymere natürlicher Herkunft, vernetzte Acrylsäure oder Methacrylsäure Homo- oder Copolymere und vernetzte Copolymere von 2-Acrylamido-2-methylpropansulfonsäure.

Ganz besondere vorteilhafte Verdicker sind Xanthangummi, wie die unter den Bezeichnungen Keltrol^{®} und Kelza^{®} von der Firma CP Kelco angebotenen Produkte oder die Produkte der Firma RHODIA mit der Bezeichnung Rhodopol^{®} und Guargummi, wie die unter der Bezeichnung Jaguar^{®} HP105 von der Firma RHODIA erhältlichen Produkte.

Ganz besondere vorteilhafte Verdicker sind weiterhin vernetzte Homopolymere von Methacrylsäure oder Acrylsäure, die von der Firma Lubrizol unter den Bezeichnungen Carbopol^{®} 940, Carbopol^{®} 941, Carbopol^{®} 980, Carbopol^{®} 981, Carbopol^{®} ETD 2001, Carbopol^{®} EDT 2050, Carbopo^{®} 2984, Carbopol^{®} 5984 und Carbopol^{®} Ultrez 10, von der Firma 3V unter den Bezeichnungen Synthalen^{®} K, Synthalen^{®} L und Synthalen^{®} MS und von der Firma PROTEX unter den Bezeichnungen Modarez^{®} V 1250 PX, Modarez^{®} V2000 PX, Viscaron^{®} A1600 PE und Viscaron^{®} A700 PE im Handel erhältlich sind.

Ganz besondere vorteilhafte Verdicker sind weiterhin vernetzte Copolymere von Acrylsäure oder Methacrylsäure und einem C₁₀₋₃₀-Alkylacrylat oder C₁₀₋₃₀-Alkyhnethacrylat und Copolymere von Acrylsäure oder Methacrylsäure und Vinylpyrrolidon. Solche Copolymere sind beispielsweise von der Firma Lubrizol unter den Bezeichnungen Carbopol^{®} 1342, Carbopol^{®} 1382, Pemulen^{®} TR1 oder Pemulen^{®} TR2 und von der Firma ISP unter den Bezeichnungen Ultrathix^{®} P-100 (INCI: Acrylic Acid/VP Crosspolymer) im Handel erhältlich.

Ganz besondere vorteilhafte Verdicker sind weiterhin vernetzte Copolymere von 2-Acrylamido-2-methylpropansulfonsäure. Solche Copolymere sind beispielsweise von der Firma Clariant unter den Bezeichnungen Aristoflex^{®} AVC (INCI: Ammonium Acryloyldimethyltaurate/VP Copolymer) erhältlich.

Diese Verdicker sind im Allgemeinen in einer Konzentration von etwa 0 % bis 2 Gew.-% vorzugsweise 0 % bis 1 Gew.-% bezogen auf das Gesamtgewicht der Zusammensetzung vorhanden.

Erfindungsgemäß vorteilhaft können die Zusammensetzungen einen oder mehrere weitere Filmbildner enthalten.

Vorteilhaft werden der oder die Filmbildner gewählt aus der Gruppe der wasserlöslichen oder wasserdispergierbaren Polyurethane, welche von den zuvor beschriebenen Polyurethanen verschieden sind, Polyharnstoffe, Polyvinylalkohole, Silikonharze und/oder Polyester sowie der nichtionischen, anionischen, amphoteren und/oder kationischen Polymere und ihren Mischungen.

Vorteilhafte nichtionische Polymere, die in erfindungsgemäßen Zusammensetzungen alleine oder im Gemisch, vorzugsweise auch mit anionischen und/oder amphoteren und/oder zwitterionischen Polymeren enthalten sein können, sind beispielsweise ausgewählt aus:
- Polyalkyloxazolinen.
- Vinylacetat Homo- oder Copolymerisate. Hierzu gehören beispielweise Copolymerisate aus Vinylacetat und Acrylsäureester, Copolymerisate aus Vinylacetat und Ethylen, Copolymerisate aus Vinylacetat und Maleinsäureester.
- Acrylsäureester Copolymerisate wie z. B. die Copolymerisate aus Alkylacrylaten und Alkylmethacrylaten, Copolymerisate aus Alkylacrylaten und (Poly)Urethanen mit entsprechender Funktionalisierung.
- Copolymerisate aus Acrylnitril und nichtionischen Monomerer, ausgewählt aus Butadien und (Meth)acrylaten.
- Styrol-Homo- und Copolymerisate. Hierzu gehören beispielweise Homopolystyrol, Copolymerisate aus Styrol und Alkyl (Meth)acrylaten, Copolymerisate aus Styrol, Alkyl-Methacrylaten und Alkyl-Acrylaten, Copolymerisate aus Styrol und Butadien, Copolymerisate aus Styrol, Butadien und Vinylpyridin.
- Polyamide.
- Polyvinylalkohole.
- Vinyllactam-Homo- oder Copolymere, wie Vinylpyrrolidon-Homo- oder Copolymerisate. Hierzu gehören beispielweise Polyvinylpyrrolidon, Polyvinylcaprolactam, Copolymerisate aus N-Vinylpyrrolidon und Vinylacetat und/oder Vinylpropionat in verschiedenen Konzentrationsverhältnissen, Copolymerisate aus Vinylpyrrolidon und einem ungesättigten Kohlenwasserstoff wie Styrol, Butadien, Hexadecen, Eicosen, Decen oder Triaconten, Polyvinylcaprolactam, Polyvinylamide und deren Salze sowie Copolymere aus Vinylpyrrolidon und Dimethylaminoethyl-methacrylat, Terpolymere aus Vinylcaprolactam, Vinylpyrrolidon und Dimethylaminoethylmethacrylat.
- Polysiloxane.
- Homopolymere des N-Vinylformamids z. B. PVF von National Starch.

Besondere bevorzugte nichtionische Polymere sind Acrylsäureester Copolymerisate, Homopolymere des Vinylpyrrolidons und Copolymere, Polyvinylcaprolactam und Polyvinylalkohol.

Ganz besondere bevorzugte nichtionische Polymere sind Homopolymere des Vinylpyrrolidons z. B. Luviskol^{®} K von der Firma BASF, Copolymerisate aus Vinylpyrrolidon und Hexadecen z.B. Antaron^{®} V-216 der Firma ISP, Copolymerisate aus Vinylpyrrolidon und Eicosen, welche unter der Handelsbezeichnung Antaron^{®} V220 bei der Firma ISP erhältlich sind, Copolymerisate aus Vinylpyrrolidon und Vinylacetat z. B. Luviskol^{®} VA Typen der Firma BASF oder PVPVA^{®} S630L der Firma ISP, Terpolymere aus Vinylpyrrolidon, Vinylacetat und Propionat wie z. B. Luviskol^{®} VAP von BASF, Polyvinylcaprolactame z.B. Luviskol^{®} PLUS der Firma BASF und Polyvinylalkohol wie z.B. Celvol^{®} 125 der Firma Celanese.

Vorteilhafte anionische Polymere sind Homo-oder Copolymere mit Säuregruppen enthaltenden Monomereinheiten, welche gegebenenfalls mit Comonomeren, die keine Säuregruppen enthalten copolymerisiert sind. Geeignete Monomere sind ungesättigte, radikalisch polymerisierbare-Verbindungen, welche mindestens eine Säuregruppe besitzen, insbesondere Carbonsäure, Sulfonsäure oder Phosphonsäure.

Vorteilhafte anionische Polymere enthaltend Carbonsäuregruppe sind:
- Acrylsäure oder Methacrylsäure-Homo- oder Copolymere oder die Salze davon. Hierzu gehören beispielweise die Copolymerisate aus Acrylsäure und Acrylamiden und/oder deren Natriumsalze, Copolymerisate aus Acrylsäure und/oder Methacrylsäure und einem ungesättigten Monomer ausgewählt aus Ethylen, Styrol, Vinylestern, Acrylsäureestern, Methacrylsäureestern, gegebenenfalls ethoxylierten Verbindungen, Copolymerisate aus Vinylpyrrolidon, Acrylsäure und C1-C20 Alkyl-Methacrylaten, z.B. Acrylidone^{®} LM der Firma ISP, Copolymerisate aus Methacrylsäure, Ethylacrylate und Tert.-Butyl Acrylate z.B. Luvimer^{®} 100 P der Firma BASF.
- Crotonsäurederivate-Homo- oder Copolymere oder die Salze davon. Hierzu gehören beispielweise Vinylacetat/Crotonsäure-, Vinylacetat/Acrylat- und/oder Vinylacetat/Vinylneodecanoal/Crotonsäure-Copolymere, Natriumacrylat/Vinylalkohol-Copolymere.
- ungesättigte C4-C8 Carbonsäurederivate oder Carbonsäureanhydrid-Copolymere ausgewählt aus Copolymerisaten aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Vinylestern, Vinylethern, Vinylhalogenderivaten, Phenylvinylderivaten, Acrylsäure, Acrylsäureestern oder Copolymerisaten aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Allylestern, Methallylestern und gegebenenfalls Acrylamiden, Methacrylamiden, alpha-Olefinen, Acrylsäureestern, Methacrylsäureestern, Vinylpyrrolidonen. Weitere bevorzugte Polymere sind Methylvinylether/Maleinsäure-Copolymere, die durch Hydrolyse von Vinylether/Maleinsäureanhydrid-Copolymeren entstehen. Diese Polymere können auch teilverestert (Ethyl, Isopropyl- bzw. Butylester) oder teilamidiert sein.
- in Wasser lösliche oder dispergierbare anionische Polyurethane, z. B. Luviset® PUR von BASF, wobei diese Aufstellung nicht limitierend ist.

Vorteilhafte anionische Polymere enthaltend Sulfonsäuregruppe sind Salze von Polyvinylsulfonsäure, Salze von Polystyrolsulfonsäure wie z. B. Natriumpolystyrolsulfonat oder Salze von Polyacrylamidsulfonsäure.

Besondere vorteilhafte anionische Polymere sind Acrylsäure-Copolymere, Crotonsäurederivat-Copolymere, Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Vinylester, Vinylether, Vinylhalogenderivate, Phenylvinylderivate, Acrylsäure, Acrylsäureester und Salze von Polystyrolsulfonsäure.

Ganz besondere vorteilhafte anionische Polymere sind Acrylat/Octylacrylamid-Copolymere, z.B. Dermacryl^{®} 79 von National Starch, Acrylat-Copolymere, z.B. Luvimer^{®} von BASF, Ethylacrylat/N-tert.-Butylacrylamid/Acrylsäure-Copolymere, wie ULTRAHOLD^{®} STRONG der Firma BASF, VA/Crotonat/Vinylneodecanoat- Copolymer z. B. Resin 28-2930 von National Starch, Copolymerisate wie zum Beispiel Copolymerisate aus Methylvinylether und Maleinsäureanhydrid teilverestert, wie z.B. GANTREZ^{®} der Firma ISP und Sodium Polystyrene Sulfonate z. B. Flexan^{®} 130 von National Starch.

Gegebenenfalls einsetzbar aber jedoch weniger bevorzugt sind kationische und amphomere Filmbildner. Beispiele sind kationische Filmbildner-Polymere, die primäre, sekundäre tertiäre und/oder quatäre Aminogruppen enthalten, die Teil der Polymerkette oder direkt an die Polymerkette gebunden sind. Vorteilhafte amphotere Filmbildner können unter den Polymeren ausgewählt werden, die statistisch in der Polymerkette verteilte Einheiten A und B enthalten, wobei A eine Einheit bedeutet, die von einem Monomer mit mindestens einem basischen Stickstoffatom abgeleitet ist, und B eine Einheit ist, die von einem sauren Monomer stammt, das eine oder mehrere Carboxygruppen oder Sulfonsäuregruppen aufweist, oder A und B können Gruppen bedeuten, die von zwitterionischen Carboxybetainmonomeren oder Sulfobetainmonomeren abgeleitet sind; A und B können auch eine kationische Polymerkette bedeuten, die primäre, sekundäre, tertiäre oder quartanäre Gruppen enthält, worin mindestens eine Aminogruppe eine Carboxygruppe oder Sulfonsäuregruppe trägt, die über eine Kohlenwasserstoffgruppe gebunden ist, oder B und C sind Teil einer Polymerkette mit Ethylen-α,β-dicarbonsäureeinheit, bei der die Carbonsäuregruppen mit einem Polyamin umgesetzt wurden, das eine oder mehrere primäre oder sekundäre Aminogruppen enthält.

Erfindungsgemäß besonders vorteilhaft ist die Konzentration eines weiteren Filmbildners 0 bis 20 Gewichts-% und insbesondere 0 bis 10 Gewichts-% jeweils bezogen auf das Gesamtgewicht der kosmetischen Zusammensetzung. Besonders bevorzugt ist die Kombination mit Polyvinylalkohol.

Es ist gegebenenfalls vorteilhaft, die anionischen und amphoteren Polymere zur Verbesserung ihrer Wasserlöslichkeit bzw. ihrer Wasserdispergierbarkeit mit geeigneten Basen zu neutralisieren.

Als Neutralisationsmittel für Polymere die Säuregruppen enthalten können folgende Stoffe eingesetzt werden: Hydroxide, deren Kation ein Ammonium oder ein Alkalimetall ist wie z. B. NaOH oder KOH.

Andere Neutralisationsmittel sind primäre, sekundäre oder tertiäre Amine, Aminoalkohole oder Ammoniak. Bevorzugt werden hier 2-Amino-2-methyl-1,3-propandiol (AMPD), 2-Amino-2-ethyl-1,3-propandiol (AEPD), 2-Amino-2-methyl-1-propanol (AMP), 2-Amino-1-butanol (AB), 2-Amino-1,3-propandiol, Monoethanolamin (MEA), Diethanolamin (DEA), Triethanolamin (TEA), Monoisopropanolamin (MIPA), Diisopropanolamin (DIPA), Triisopropanolamin (TIPA), Dimethyl Laurylamin (DML), Dimethyl Myristalamin (DMM), und Dimethyl Stearamin (DMS).

Die Neutralisation kann je nach Anwendungszweck teilweise oder vollständig erfolgen.

Die erfindungsgemäße kosmetische Zusammensetzung, welche insbesondere eine Zusammensetzung zum Auftrag auf die Haut, insbesondere die Gesichtshaut darstellt, enthält zweckmäßig einen oder mehrere kosmetisch wirksame, gegebenenfalls auch pharmazeutisch wirksamen Inhaltsstoffe, insbesondere solche Wirkstoffe, die eine reinigende und/oder pflegende Wirkung auf die Haut, insbesondere auf die Gesichtshaut aufweisen. Die kosmetischen Zusammensetzungen der Erfindung sind im wesentlichen Mittel im Sinne des Lebensmittel- und Bedarfsgegenständegesetzes (LMBG), d.h., Stoffe oder Zubereitungen aus Stoffen, die dazu bestimmt sind, äußerlich am Menschen zur Reinigung, Pflege, oder zur Beeinflussung des Aussehens oder des Körpergeruchs, oder zur Vermittlung von Geruchseindrücken angewendet zu werden, es sei, denn, dass sie überwiegend dazu bestimmt sind, Krankheiten, Leiden, Körperschäden oder krankhafte Beschwerden zu lindern oder zu beseitigen. In diesem Sinne handelt es sich bei den erfindungsgemäßen kosmetischen Zusammensetzungen insbesondere um Mittel zum Auftrag auf die Haut, insbesondere um reinigende und/oder pflegende kosmetische Auflagen, insbesondere für die Gesichthaut, insbesondere als abziehbare, filmbildende Reinigungsmaske, wie insbesondere eine Gesichtsmaske. Abziehbar bedeutet im Allgemeinen, dass die Maske in einem Stück abziehbar ist (zusammenhängender Film).

Daher enthält sie insbesondere dermatologisch wirksameWirkstoffe, wie reinigende Wirkstoffe und/oder Hautpflegemittel, insbesondere Gesichtshautpflegemittel.

Beispiele kosmetisch gegebenenfalls auch therapeutisch wirksamer Inhaltsstoffe schließen ein: Antiaknemittel, antimikrobielle Mittel, Antitranspirationsmittel, adstringierende Mittel, desodorierende Mittel, Konditionierungsmittel für die Haut, hautglättende Mittel, Mittel zur Steigerung der Hauthydratation wie z. B. Glycerin oder Harnstoff, Keratolytika, Radikalfänger für freie Radikale, antiseptische Wirkstoffe, Wirkstoffe gegen Hautalterung und/oder Mittel, welche die Differenzierung und/oder Proliferation und/oder Pigmentierung der Haut modulieren, Vitamine wie Vitamin C, Wirkstoffe mit reizender Nebenwirkung, wie alpha-Hydroxysäuren, β-Hydroxysäuren, alpha-Ketosäuren, β-Ketosäuren, Retinoide (Retinol, Retinal, Retin-Säure) Anthralinen (Dioxyanthranol), Anthranoide, Peroxide (insbesondere Benzoylperoxid), Minoxidil, Lithiumsalze, Antimetabolite, Vitamin D und seine Derivate; Katechine, Flavonoide, Ceramide, Fettsubstanzen, synthetische Öle, Mineralöle, wie Paraffinöle oder Vaselineöle, Siliconöle, Pflanzenöle wie Kokosöl, Süßmandelöl, Aprikosenöl, Maisöl, Jojobaöl, Olivenöl, Avocadoöl, Sesamöl, Palmöl, Eukalyptusöl, Rosmarinöl, Lavendelöl, Kiefernöl, Thymianöl, Minzöl, Kardamonöl, Orangenblütenöl, Sojaöl, Kleieöl, Reisöl, Rapsöl und Rizinusöl, Weizenkeimöl und daraus isoliertes Vitamin E, Nachtkerzenöl, Pflanzenlecithine (z.B. Sojalecithin), aus Pflanzen isolierte Sphingolipide/Ceramide, tierische Öle oder Fette, wie Talg, Lanolin, Butteröl, Fettsäureester, Ester von Fettalkoholen und Wachse mit einem der Hauttemperatur entsprechenden Schmelzpunkt (tierische Wachse, wie Bienenwachs, Carnaubawachs und Candelillawachs, mineralische Wachse, wie mikrokristalline Wachse, und synthetische Wachse, wie Polyethylen- oder Silikonwachse), sowie sämtliche für kosmetische Zwecke geeigneten Öle, wie beispielsweise in der CPCP-Abhandlung, International Cosmetic Ingredient Dictionary Handbook, 12. Auflg., 2008, The Personal Care Product Council (früher The Cosmetic, Toiletry and Fragrance Association), Inc., Washington, erwähnt, mehrfach ungesättigte Fettsäuren, essentielle Fettsäuren (z.B. gamma-Linolensäure), Enzyme, Coenzyme, Enzymhemmstoffe, hydratisierende Mittel, hautberuhigende Mittel, Detergentien oder schaumbildende Mittel, und anorganische oder synthetische mattierende Füllstoffe, mit Ausnahme der nicht vorteilhaften oben genannten nicht-verfihnenden Füllstoffe, abrasive Mittel.

So enthalten die erfindungsgemäßen kosmetischen Zusammensetzung bevorzugt weniger als 20 Gew.-%, bevorzugter weniger als 10 Gew.-%, noch bevorzugter weniger als 1 Gew.-% des nicht-filmbildenden Füllstoffs Kaolin, bezogen auf das Gewicht der kosmetischen Zusammensetzungen.

Weiterhin enthalten die erfindungsgemäßen kosmetischen Zusammensetzung von jedem einzelnen der nicht-filmbildenden Füllstoffe, die aus der Gruppe ausgewählt sind, die besteht aus: Kieselsäure, Talk, Tonen, Titandioxid, Zinkoxid, Mikrokugeln, Stärke, modifizierte Stärkeverbindungen bevorzugt weniger als 20 Gew.-%, bevorzugter weniger als 10 Gew.-% und noch bevorzugter weniger als 1 Gew.-%, bezogen auf das Gewicht der kosmetischen Zusammensetzungen. Bevorzugter enthalten die erfindungsgemäßen kosmetischen Zusammensetzung in Summe von den Füllstoffen, die aus der Gruppe ausgewählt sind, die besteht aus: Kieselsäure, Talk, Tonen, Titandioxid, Zinkoxid, Mikrokugeln, Stärke, modifizierte Stärkeverbindungen bevorzugt weniger als 20 Gew.-%, bevorzugter weniger als 10 Gew.-% und noch bevorzugter weniger als 1 Gew.-%, bezogen auf das Gewicht der kosmetischen Zusammensetzungen.

Weiterhin enthalten die erfindungsgemäßen kosmetischen Zusammensetzung bevorzugt weniger als 20 Gew.-%, bevorzugter weniger als 10 Gew.-%, noch bevorzugter weniger als 1 Gew.-% nicht-filmbildender Füllstoffe Kaolin, bezogen auf das Gewicht der kosmetischen Zusammensetzungen.

Besondere bevorzugt enthalten die kosmetischen Zusammensetzung keinerlei (< 1 Gew.-%) nicht-filmbildende Füllstoffe, wie insbesondere Kieselsäure, Talk, Tonen, wie Kaolin, Titandioxid, Zinkoxid, Mikrokugeln, Stärke oder modifizierte Stärkeverbindungen.

Nicht-filmbildende Füllstoffe sind solche, die beim Trocknen aus wässrigen Dispersionen keine zusammenhängende Filme bilden.

Weiterhin können die erfindungsgemäßen kosmetischen Zusammensetzungen Pflanzenwirkstoffextrakte bzw. daraus gewonnene Auszüge oder Einzelstoffe erwähnt werden enthalten sein, wie solche, die ausgewählt werden aus der Gruppe bestehend aus festen Pflanzenextrakten, flüssigen Pflanzenextrakten, hydrophilen Pflanzenextrakten, lipophilen Pflanzenextrakten, einzelnen Pflanzeninhaltsstoffen; sowie deren Mischungen, wie Flavonoide und ihre Aglyka: Rutin, Quercetin, Diosmin, Hyperosid, (Neo)hesperidin, Hesperitin, Ginkgo Biloba (z.B. Ginkoflavonglykoside), Crataegus-Extrakt (z.B. oligomere Procyanidine), Buchweizen (z.B. Rutin), Sophora japonica (z.B. Rutin), Birkenblätter (z.B. Quercetinglykoside, Hyperosid und Rutin), Holunderblüten (z.B. Rutin), Lindenblüten (z.B. ätherisches Öl mit Quercetin und Farnesol), Johanniskrautöl, (z.B. Olivenölauszug), Calendula, Arnika (z.B. ölige Auszüge der Blüten mit ätherischem Öl, polare Auszüge mit Flavonoiden), Melisse (z.B. Flavone, ätherisches Öl); Immunstimulantien: Echinacea purpurea (z.B. alkoholische Auszüge, Frischpflanzensaft, Preßsaft), Eleutherokokkus senticosus; Alkaloide: Rauwolfia (z.B. Prajmalin), lmmergrün (z.B.Vincamin); weitere Phytopharmaka: Aloe, Roßkastanie (z.B. Aescin), Knoblauch (z.B. Knoblauchöl), Ananas (z.B. Bromelaine), Ginseng (z.B. Ginsenoside), Mariendistelfrüchte (z.B. auf Silymarin standardisierter Extrakt), Mäusedornwurzel (z.B. Ruscogenin), Baldrian (z.B. Valepotriate, Tct. Valerianae), Kava-Kava (z.B. Kavalactone), Hopfenblüten (z.B. Hopfenbitterstoffe), Extr. Passi-florae, Enzian (z.B. ethanol. Extrakt), anthrachinonhaltige Drogenauszüge, z.B. aloinhaltiger Aloe Vera-Saft, Pollenextrakt, Algenextrakte, Süßholzwurzelextrakte, Palmenextrakt, Galphimia (z.B.Urtinktur), Mistel (z.B. wässrig-ethanol. Auszug), Phytosterole (z.B. beta-Sitosterin), Wollblumen (z.B. wäßrig-alkohol. Extrakt), Drosera (z.B. Likörweinextrakt), Sanddornfrüchte (z.B. daraus gewonnener Saft oder Sanddornöl), Eibischwurzel, Primelwurzelextrakt, Frischpflanzenextrakte aus Malve, Beinwell, Efeu, Schachtelhalm, Schafgarbe, Spitzwegerich (z.B. Preßsaft), Brennessel, Schöllkraut, Petersilie; Pflanzenextrakte aus Norolaena lobata, Tagetes lucida, Teeoma siems, Momordica charantia, und Aloe Vera Extrakte. Bevorzugte kosmetische Wirkstoffe sind natürliche und synthetische Feuchthaltefaktoren bzw. Feuchthaltemittel, wie beispielsweise Glycerin, Polyglycerin, Sorbit, Dimethylisosorbid, Milchsäure und/oder Lactate, insbesondere Natriumlactat, Butylenglykol, Propylenglykol, Biosaccaride Gum-1, Glycine Soja, Hydroxyethylharnstoff, Ethylhexyloxyglycerin, Pyrrolidoncarbonsäure und Harnstoff, polymere Moisturizer aus der Gruppe der wasserlöslichen und/oder in Wasser quellbaren und/oder mit Hilfe von Wasser gelierbaren Polysaccharide, Hyaluronsäure, Chitosan, fucosereiche Polysaccharide, welche unter der Bezeichnung Fucogel™ 1000 von der Gesellschaft SOLABIA S.A. erhältlich sind, des weiteren Ceramide, Hautschutzmittel, Hautaufheller, Vitamine, Antioxidantien, sogenannte Antiagingmittel, antiirritative Mittel etc. Weitere bevorzugte kosmetische Wirkstoffe sind natürliche Fette und Öle, d.h. Triglyceride von natürlichen Fettsäuren, beispielsweise aufgrund ihrer rückfettenden und pflegenden Wirkung auf der Haut.

Besonders vorteilhaft im Sinne der vorliegenden Erfindung können wasserlösliche Antioxidantien eingesetzt werden, wie beispielsweise Vitamine, z.B. Ascorbinsäure und deren Derivate. Ganz besonders vorteilhafte sind Vitamin E und dessen Derivate sowie Vitamin A und dessen Derivate.

Weitere vorteilhafte Wirkstoffe in der erfindungsgemäßen Zusammensetzung sind α-Hydroxysäure wie Glykolsäure, Milchsäure, Äpfelsäure, Weinsäure, Citronensäure und Mandelsäure, β-Hydroxysäure wie Salicylsäure sowie deren acylierten Derivate, die 2-Hydroxyalkansäure und ihre Derivate; natürliche Wirkstoffe und/oder deren Derivate, wie z.B. alpha-Liponsäure, Folsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder [beta]-Alanin sowie 8-Hexadecen-1,16-dicarbonsäure (Dioic acid, CAS-Nummer 20701-68-2; vorläufige INCI-Bezeichnung Octadecendioic acid) und/oder Licochalcon A und die Pflanzenextrakte.

Pharmazeutische bzw. therapeutische Wirkstoffe sind solche, die im Sinne des Arzneimittelgesetzes unter anderem dazu bestimmt sind, Krankheiten, Leiden, Körperschäden oder krankhafte Beschwerden zu heilen, zu lindern oder zu verhüten. Die Mittel bzw. Wirkstoffe sind für die äußere Anwendung bestimmt, wobei es sich um hautaktive Wirkstoffe aber auch um transdermale Wirkstoffe handelt kann. Sie schließen beispielsweise ein: Mittel zur Behandlung von Hautkrankheiten, wie antibakterielle Wirkstoffe, Antimykotika, antivirale Wirkstoffe, entzündungshemmende Wirkstoffe, wie Dexpanthenol, juckreizstillende Wirkstoffe, Cortison und Derivate, wie Glukokortikoide, wie Prednison, Prednisolon, Methylprednisolon, Betamethason, Dexamethason, Triamcinolon, Paramethason und Fludrocortison, Mittel zur Behandlung von Hautkrankheiten, wie der Neurodermitis, der atopischen Dermatitis etc., und Anti-Herpesmittel.

Erfindungsgemäß besonders vorteilhaft enthalten die kosmetischen Zusammensetzungen ein oder mehrere Öle, wie die vorstehend erwähnten Öle, beispielweise Öle pflanzlicher Herkunft wie Aprikosenkernöl, Perhydrosqualen, Avocadoöl, Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl und dergleichen mehr; Mineralöle wie Vaselineöl; synthetische Öle wie hydriertes Polyisobuten, Octyldodecanol, Ester von Fettsäuren und Fettalkoholen mit 6 bis 30 Kohlenstoffatomen, gesättigte oder verzweigte Fettalkoholether mit 4 bis 30 Kohlenstoffatomen, Silikonöle, insbesondere flüchtige Siliconöl, wie Cyclomethicone, fluorierte Öle und deren Mischung.

Besondere vorteilhaft sind die Öle pflanzlicher Herkunft und die synthetischen Öle.

Ganz besondere vorteilhafte sind die Öle: Aprikosenkemöl, Squalan, Perhydrosqualen, Avocadoöl, hydriertes Polyisobuten und Octyldodecanol.

Die Ölmenge kann im Bereich von 0 bis 30 Gew.%, vorzugsweise 0 bis 20 Gew.%, besondere vorzugsweise von 0 bis 10 Gew.%, bezogen auf das Gesamtgewicht der kosmetischen Zusammensetzung, liegen. Bevorzugt sind jedoch keine Öle enthalten.

Die Mengen der verschiedenen, insbesondere kosmetischen Wirkstoffe sind dem Fachmann bekannt und liegen beispielweise im Bereich von 0 bis 35 Gew. % bezogen auf das Gesamtgewicht der kosmetischen Zusammensetzung.

Die erfindungsgemäße kosmetische Zusammensetzung insbesondere zur Herstellung der Reinigungs- bzw. Gesichtsmasken, enthaltend die Polyurethandispersion, kann zusätzliche übliche Hilfs- und/oder Zusatzstoffe enthalten, die in der Kosmetik üblich sind, wie Stabilisatoren, wie Antioxidantien, Lichtschutzmittel, Emulgatoren, grenzflächenaktive Stoffe, Entschäumer, Verdicker, Tenside (anionisch, kationisch oder nicht-ionisch), Lösemittel, Koaleszenzmittel, Aromastoffe, Geruchsabsorber, Parfüms, Gelbildner, andere Polymerdispersionen wie beispielsweise Dispersionen auf Basis von Polyacrylaten enthalten, Füllstoffe, Weichmacher, Pigmente, Farbstoffe, Verlaufsmittel und/oder Thixotropiemittel, Geschmeidigkeitsmittel, Weichmacher, Konservierungsmittel, pH-Einstellungsmittel, wie Pufferstoffe, Schmiermittel, Gleitmittel, etc. Die Mengen der verschiedenen Hilfs- und Zusatzstoffe sind dem Fachmann für den einzusetzenden Bereich bekannt und liegen beispielweise im Bereich von 0 bis 25 Gew. % bezogen auf das Gesamtgewicht der Zusammensetzung.

Das genannte Geschmeidigkeitsmittel bringen Geschmeidigkeit ein, wenn die Maske abgezogen wird. Die bevorzugten Geschmeidigkeitsmittel sind beispielweise ethoxylierte Polydimethylsiloxane, Dimethylisosorbid oder deren Gemische.

Die bevorzugten Weichmacher werden beispielweise ausgewählt aus der Gruppe, die besteht aus: Diisobutyladipat, Erster von t-Butansäure und 2,2,4-Trimethylpentan-1,3-diol, Diethyladipat, Diethylphtalate, Dibutylphthalat, Diocylphthalat, Butyl-2-ethylhexylphthalat, Dimethylsebacat, Dibutyldebacat, Ethylstearat, 2-ethylhexylpalmitat, Dipropylenglykoldimethylether und deren Mischung.

Die erfindungsgemäßen kosmetischen Zusammensetzungen sind insbesondere als Hautreinigungs- und/oder Hautpflege-Zusammensetzung geeignet. Insbesondere dienen sie als Maske für die Reinigung der Haut, insbesondere als filmbildende, abziehbare bzw. abschälbare Maske für die Reinigung der Haut. Derartige Hautreinigungsmasken (auch "Schönheitsmasken") sind dem Fachmann an sich bekannt (s. z.B.: "Cosmetic and Toiletry Formulations", 2. Auflage, Ernest W. Flick 1992). Ihre Anwendung erfolgt dabei durch Auftragen der kosmetischen Zusammensetzung auf die Haut, insbesondere die Gesichtshaut. Die Maskenzusammensetzung ist demnach leicht verteilbar. Nach dem Auftrag auf die Haut lässt man die frisch aufgetragene Maske einwirken. Während der Einwirkzeit wirken die kosmetisch oder therapeutisch wirksamen Inhaltsstoffe auf die Haut oder transdermal ein und bilden dabei im Falle der erfindungsgemäß bevorzugten abziehbaren bzw. abschälbaren Maske einen zusammenhängenden Film (ein Stück) aus, der das Abnehmen der kosmetischen Zusammensetzung naturgemäß erheblich erleichtert. Mit dem Abnehmen werden gleichzeitig die während der Einwirkzeit durch die Maske aufgenommenen Verunreinigungen der Haut, wie Staub, Fette, Bakterien, Bakterienstoffwechselprodukte, abgestorbene Hautzellen etc. entfernt (gelegentlich auch als Peeling-Effekt bezeichnet). Erfindungsgemäß lässt sich die Gesichtmaske aufgrund der vorteilhaften Eigenschaften der verwendeten besonderen Polyurethane(-Dispersionen) besonders gut, d.h. schonend und leicht abziehen. Vorteile der erfindungsgemäßen kosmetischen Gesichtsmaske sind insbesondere die Möglichkeit einer schnellen Trocknung sowie einer schnellen Abziehbarkeit nach Auftrag, die Abziehbarkeit in einem Stück, kein oder ein geringes Schmerzempfinden beim Abziehen, eine sehr gute Reinigungswirkung sowie eine geringe Gesichtsrötung. Die Trockungszeiten der erfindungsgemäßen, abziehbaren Masken können beispielsweise bei etwa 5 bis 30 Minuten, bevorzugt 10 bis 20 Minuten liegen. Die erfindungsgemäßen Reinigungsmasken werden vorteilhaft als Serum, Gel, Dispersion, Suspension oder Emulsion verwendet.

Die vorliegende Erfindung wird an Hand von Beispielen erläutert, wobei sie nicht als einschränkend zu verstehen sind. Alle Mengenangaben, Anteile und Prozentanteile sind, soweit nicht anders angegeben, auf das Gewicht und die Gesamtmenge bzw. auf das Gesamtgewicht der Zusammensetzungen bezogen.

### Bevorzugte Ausführungsformen der Erfindung sind insbesondere Folgende:

1. Kosmetische Zusammensetzung zum Auftrag auf die Haut, enthaltend ein oder mehrere Polyurethane, wobei genannte Polyurethane mindestens eine Sequenz enthalten, die ausgewählt wird aus der Gruppe, die besteht aus: Polyether-Sequenzen, Polycarbonat-Sequenzen und Polyether-Polycarbonat-Sequenzen.
2. Kosmetische Zusammensetzung nach Ausführungsform 1, die filmbildende Eigenschaften hat.
3. Kosmetische Zusammensetzung nach Ausführungsform 1 oder 2, worin das Polyurethan eine Reißdehnung von mindestens 500 % aufweist.
4. Kosmetische Zusammensetzung nach einer der Ausführungsformen 1 bis 3, worin das Polyurethan dispergiert vorliegt.
5. Kosmetische Zusammensetzung nach Ausführungsform 4, worin das Polyurethan in wässriger Dispersion vorliegt.
6. Kosmetische Zusammensetzung nach einer der Ausführungsformen 1 bis 5, worin das Polyurethan erhältlich ist unter Verwendung von polymeren Polyether-Polyolen und/oder polymeren Polycarbonat-Polyolen und/oder Polyether-Polycarbonat-Polyolen welche jeweils zahlenmittlere Molekulargewichte von 400 bis 8000 g/mol aufweisen.
7. Kosmetische Zusammensetzung nach Ausführungsform 6, worin die polymeren Polyether-Polyole, die Polycarbonat-Polyole und die Polyether-Polycarbonat-Polyole jeweils Diole mit linearem Aufbau sind.
8. Kosmetische Zusammensetzung nach einer der Ausführungsformen 1 bis 7, worin das Polyurethan Polycarbonat-Sequenzen enthält.
9. Kosmetische Zusammensetzung nach einem der Ausführungsformen 1 bis 8, worin das Polyurethan Polyether-Sequenzen enthält.
10. Kosmetische Zusammensetzung nach Ausführungsform 9, worin die Polyether-Sequenz eine Polytetramethylenglykol-Sequenz ist.
11. Kosmetische Zusammensetzung nach einer der Ausführungsformen 1 bis 10, worin das Polyurethan mindestens eine anionische Gruppe oder potentiell anionische Gruppe aufweist.
12. Kosmetische Zusammensetzung nach Ausführungsform 11, worin die anionische Gruppe eine Sulfonat-Gruppe ist.
13. Kosmetische Zusammensetzung nach einer der Ausführungsformen 1 bis 12, welche 5 bis 90 Gew.-% einer wässrigen Dispersion des Polyurethans enthält.
14. Kosmetische Zusammensetzung nach einer der Ausführungsformen 1 bis 13, welche 1 bis 90 Gew.-%, bevorzugt mehr als 30 Gew.-% des Polyurethans, bezogen auf den Festkörpergehalt der kosmetischen Zusammensetzung enthält.
15. Kosmetische Zusammensetzung nach einer der Ausführungsformen 1 bis 14, welche weniger als 1 Gew.-% Kaolin enthält.
16. Kosmetische Zusammensetzung nach einer der Ausführungsformen 1 bis 15, welche in Summe weniger als 1 Gew.-% Füllstoffe enthält, die aus der Gruppe ausgewählt sind, die besteht aus: Kieselsäure, Talk, Tonen, Titandioxid, Zinkoxid, Mikrokugeln, Stärke, modifizierte Stärkeverbindungen.
17. Kosmetische Zusammensetzung nach einer der Ausführungsformen 1 bis 16, welche weniger als 1 Gew.-%, nicht-filmbildender Füllstoffe enthält.
18. Kosmetische Zusammensetzung nach einer der Ausführungsformen 1 bis 17, welche keine nicht-filmbildenden Füllstoffe enthält.
19. Kosmetische Zusammensetzung nach einer der Ausführungsformen 1 bis 18 zum Auftrag auf die Gesichtshaut.
20. Kosmetische Zusammensetzung nach einer der Ausführungsformen 1 bis 19 zur Hautreinigung und/oder Hautpflege.
21. Kosmetische Zusammensetzung nach einer der Ausführungsformen 1 bis 20 zur Herstellung einer Gesichtsmaske.
22. Kosmetische Zusammensetzung nach Ausführungsform 21 zur Herstellung einer abziehfähigen Gesichtsmaske.
23. Kosmetische Zusammensetzung nach einer der Ausführungsformen 1 bis 22, enthaltend mindestens einen kosmetisch und/oder therapeutisch wirksamen Inhaltsstoff.
24. Verfahren zur Herstellung der kosmetischen Zusammensetzung nach einer der Ausführungsformen 1 bis 23, welches den Schritt des Vermischens einer wässrigen Dispersion des genannten Polyurethans mit mindestens einem kosmetisch und/oder therapeutisch wirksamen Inhaltsstoff umfasst.
25. Verwendung einer kosmetischen Zusammensetzung nach einer der Ausführungsformen 1 bis 23 zum Auftrag auf die Haut, bevorzugt die Gesichtshaut.
26. Kosmetisches Verfahren zur Reinigung und/oder Pflege der Haut, welches den Auftrag der Zusammensetzung nach einer der Ausführungsformen 1 bis 23 auf die Haut, bevorzugt die Gesichtshaut, und das anschließende Entfernen der Zusammensetzung von der Haut, bevorzugt der Gesichtshaut, umfasst.
27. Kosmetisches Verfahren nach Ausführungsform 26, worin das Entfernen der Zusammensetzung von der Haut, bevorzugt die Gesichtshaut, durch Abziehen der getrockneten Zusammensetzung in einem Stück erfolgt.
28. Verwendung eines Polyurethans, welches eine Reißdehnung von mindestens 500 % aufweist, zur Herstellung kosmetischer Zusammensetzungen zum Auftrag auf die Haut, bevorzugt die Gesichtshaut.

### Beispiele:

Sofern nicht abweichend gekennzeichnet, beziehen sich alle Prozentangaben auf das Gewicht.

Die Bestimmung der Festkörpergehalte erfolgte nach DIN-EN ISO 3251.

NCO-Gehalte wurden, wenn nicht ausdrücklich anders erwähnt, volumetrisch gemäß DIN-EN ISO 11909 bestimmt.

Sofern nicht abweichend vermerkt, beziehen sich alle analytischen Messungen auf Temperaturen von 23°C.

Die angegebenen Viskositäten wurden mittels Rotationsviskosimetrie nach DIN 53019 bei 23°C mit einem Rotationsviskosimeter der Firma Anton Paar Germany GmbH, Ostfildern, DE bestimmt.

Die Festkörpergehalte wurden ermittelt durch Erhitzen einer ausgewogenen Probe auf 125°C. Bei Gewichtskonstanz wurde durch erneutes Auswiegen der Probe der Festkörpergehalt berechnet.

Die Kontrolle auf freie NCO-Gruppen wurde mittels IR-Spektroskopie (Bande bei 2260 cm⁻¹) durchgeführt.

Die Bestimmung der mittleren Teilchengrößen (angegeben ist das Zahlenmittel) der Polyurethan-Dispersionen erfolgte mittels Laserkorrelations-Spektroskopie nach Verdünnung mit entionisiertem Wasser (Gerät: Malvem Zetasizer 1000, Malver Inst. Limited).

### Verwendete Substanzen und Abkürzungen:

- Diaminosulfonat:: NH₂-CH₂CH₂-NH-CH₂CH₂-SO₃Na (45 %ig in Wasser)
- Desmophen^{®} C2200:: Polycarbonatpolyol, OH-Zahl 56 mg KOH/g, zahlenmittleres Moleku- largewicht 2000 g/mol (BayerMaterialScience AG, Leverkusen, DE)
- PolyTHF^{®} 2000:: Polytetramethylenglykolpolyol, OH-Zahl 56 mg KOH/g, zahlenmitt- leres Molekulargewicht 2000 g/mol (BASF AG, Ludwigshafen, DE)
- PolyTHF^{®} 1000:: Polytetramethylenglykolpolyol, OH-Zahl 112 mg KOH/g, zahlen- mittleres zahlenmittleres Molekulargewicht 1000 g/mol (BASF AG, Ludwigshafen, DE)

- Polyether LB 25:: monofunktioneller Polyether auf Ethylenoxid-/Propylenoxidbasis, zahlenmittleres Molekulargewicht 2250 g/mol, OH-Zahl 25 mg KOH/g (BayerMaterialScience AG, Leverkusen, DE)
- Impranil^{®} XP 2611: Anionisch hydrophilierte, aliphatische Polyesterpolyurethan- Dispersion ohne Polyetherbausteine und Polycarbonatbausteine, in Wasser mit einem Feststoffgehalt von ca. 40 Gew.-%, Bayer MaterialScience AG, Leverkusen, DE

Die weiteren Chemikalien wurden vom Chemikalienfachhandel bezogen (Sigma-Aldrich Chemie GmbH, Taufkirchen, DE).

### Beispiel 1: PUR-Dispersion

987,0 g Desmophen^{®} C2200 (Komponente A2a)), 761,3 g PolyTHF^{®} 2000 (Komponente A2a)), 375,4 g PolyTHF^{®} 1000 (Komponente A2a)) und 53,2 g Polyether LB 25 (Komponente A4)) wurden auf 70°C aufgeheizt. Anschließend wurde bei 70°C innerhalb von 5 min ein Gemisch aus 237,0 g Hexamethylendiisocyanat (Komponente A1)) und 313 g Isophorondiisocyanat (Komponente A1)) zugegeben und solange unter Rückfluss gerührt bis der theoretische NCO-Wert unterschritten war. Das fertige Prepolymer wurde mit 4850 g Aceton bei 50°C gelöst und anschließend eine Lösung aus 25,1 g Ethylendiamin (Komponente B1)), 61,7 g Diaminosulfonat (Komponente B2)) und 116,5 g Isophorondiamin (Komponente B1)) und 1030 g Wasser innerhalb von 10 min zudosiert. Die Nachrührzeit betrug 10 min. Danach wurde innerhalb von 10 min durch Zugabe von 1080 g Wasser dispergiert. Es folgte die Entfernung des Lösemittels durch Destillation im Vakuum und es wurde eine lagerstabile Dispersion mit einem Festkörpergehalt von 60,0 % erhalten.

| | |
|---|---|
| Feststoffgehalt: | 60 % |
| Partikelgröße (LKS): | 278 nm |
| Viskosität | 220 mPas |

### Beispiel 2: PUR-Dispersion

450 g PolyTHF^{®} 1000 (Komponente A2a)) und 2100 g PolyTHF^{®} 2000 (Komponente A2a)) wurden auf 70°C aufgeheizt. Anschließend wurde bei 70°C innerhalb von 5 min ein Gemisch aus 225,8 g Hexamethylendiisocyanat (Komponente A1)) und 298,4 g Isophorondiisocyanat (Komponente A1)) zugegeben und solange bei 100-115°C gerührt bis der theoretische NCO-Wert unterschritten war. Das fertige Prepolymer wurde mit 5460 g Aceton bei 50°C gelöst und anschließend eine Lösung aus 29,5 g Ethylendiamin (Komponente B1)), 143,2 g Diaminosulfonat (Komponente B2)) und 610 g Wasser innerhalb von 10 min zudosiert. Die Nachrührzeit betrug 15 min. Danach wurde innerhalb von 10 min durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 56 % |
| Partikelgröße (LKS): | 276 nm |
| Viskosität | 1000 mPas |

### Beispiel 3: PUR-Dispersion

450 g PolyTHF^{®} 1000 (Komponente A2)) und 2100 g PolyTHF^{®} 2000 (Komponente A2)) wurden auf 70°C aufgeheizt. Anschließend wurde ein Gemisch aus 225,8 g Hexamethylendiisocyanat (Komponente A1)) und 298,4 g Isophorondiisocyanat (Komponente A1)) zugegeben und solange bei 100-115°C gerührt bis der theoretische NCO-Wert unterschritten war. Das fertige Prepolymer wurde mit 5460 g Aceton bei 50°C gelöst und anschließend eine Lösung aus 351 g Diaminosulfonat (Komponente B2)) und 610 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 42 % |
| Viskosität: | 1370 mPas |

### Anwendungstechnische Beispiele: Abziehfähige Gesichtsmaskenformulierungen:

| | A | B | C |
|---|---|---|---|
| Polyurethan aus Beispiel 2 (bezogen auf Feststoff in der Polyurethan-Dispersion) | 15 | 10 | 20 |
| Glycerin | 2 | 10 | |
| Panthenol | | 0,5 | |
| Dimethylisosorbid | | | 0,5 |
| PEG-12 dimethicone | 0,5 | | |
| PEG-57PPG-3 Methicone | | | 0,5 |
| Xanthan gum | 2,0 | | |
| Acrylate/ C10-30 Alkyl Acrylate Crosspolymer | | | 0,5 |
| Cyclopentasiloxane | | | 2 |
| Polyvinylalkohol | | 5 | |
| Ethanol | 10 | 15 | |
| Wirkstoff | q.s. | q.s. | q.s. |
| Farbstoff | q.s. | q.s. | q.s. |
| Neutralisationsmittel | q.s. | q.s. | q.s. |
| Konservierungsmittel | q.s. | q.s. | q.s. |
| Parfüm | q.s. | q.s. | q.s. |
| Wasser | ad 100 | ad 100 | ad 100 |

(Angaben in Gew.-% bezogen auf die kosmetische Zusammensetzung).

Die Reißdehnung wurde mit einer Reißmaschine der Fa. Zwick, Modell Z 1.0 durchgeführt. Die Durchführung erfolgte gemäß der DIN 53 504 "Prüfung von Kautschuk und Elastomeren". Für diese Prüfung wurden Prüfkörper der Größe S 4 ausgestanzt.

Die Prüfgeschwindigkeit wurde im Messprogramm voreingstellt und betrug 400 mm/min. Die Vorkraft betrug 0,05 N. Pro Film wurden jeweils 4 Prüfkörper gerissen.

Die Polyurethandispersion dafür wurd mit Borchi Gel ALA auf Streichviskosität (ca. 6000 mPas) eingestellt und es wurde ein 500 µm Nassfilm auf Trennpapier VEZ matt mittels eines WASAG Lineals appliziert. Der Film wurde bei 50 °C trocknen und anschießend 3 Minuten bei 150 °C gelagert.

### Vergleichversuche Peel-Off Maske

**Tabelle 1:**

| | 1 | 2 | 3 |
|---|---|---|---|
| | Polyester-Polyurethan (aus Beispiel 3)* | Polyether-Polyurethan (aus Beispiel 2)* | Polyester-Polyurethan Impranil XP 2611 (Vergleich) |
| Reißdehnung (%) | 1720 | 1450 | 300 |

| | | | |
|---|---|---|---|
| * : erfindungsgemäß | | | |

Die drei Polyurethane Filmbildner wurden in die folgenden Formulierung eingearbeitet:

**Tabelle 2:**

| Rohstoffe | Gew.% bezogen auf die kosmetische Zusammensetzung |
|---|---|
| Polyurethan (bezogen auf Feststoff in der Polyurethan-Dispersion) | 20 |
| Acrylat/C10-30 Alkylacrylat-Crosspolymer (Carbopol® 13 82 der Firma Lubrizol) | 0,3 |
| NaOH | q.s. für pH 7 |
| Wasser | Ad. 100 |

Auf ein Stück von 2,2 mal 4 cm Schweinhaut wurden 0,2 g der oben beschriebenen Formulierung homogen verteilt. Nach 30 Minuten Trocknung bei RT wurde der gebildete Film abgezogen.

**Tabelle 3:**

| Versuch | 4 * | 5 * | 6 |
|---|---|---|---|
| Dispersion entsprechend Tabelle 1 | 1 | 2 | 3 |
| | Polyester-Polyurethan (aus Beispiel 3) | Polyether-Polyurethan (aus Beispiel 2) | Polyester-Polyurethan (Vergleich) |
| Abziehbarkeit | Ausgezeichnet, in einem Stück | Ausgezeichnet, in einem Stück | schlecht |

| | | | |
|---|---|---|---|
| * : erfindungsgemäß | | | |

Es zeigte sich bei den Anwendungsbeispielen, dass eine reinigende abziehfahige Maske für das Gesicht basierend auf den erfindungsgemäßen kosmetischen Zusammensetzungen deutliche Vorteile gegenüber dem Stand der Technik bietet. Die Verwendung der erfindungsgemäßen Zusammensetzung ermöglicht einen gleichmäßigen Auftrag, eine schnelle Trocknung, mithin eine schnelle Abziehbarkeit nach dem Auftrag, eine Abziehbarkeit in einem Stück, verbunden mit einem geringen Schmerzempfinden beim Abziehen, eine sehr gute Reinigungswirkung und eine geringe Gesichtsrötung. Die Nachteile der Füllstoffe, z. Bi von Kaolin, wie eine schlechte Abziehbarkeit in einem Stück werden mittels der speziellen Polyurethane gelöst.

## Patentansprüche

1. Kosmetische Zusammensetzung zum Auftrag auf die Haut, enthaltend ein oder mehrere Polyurethane, wobei genannte Polyurethane mindestens eine Sequenz enthalten, die ausgewählt wird aus der Gruppe, die besteht aus: Polyether-Sequenzen, Polycarbonat-Sequenzen und Polyether-Polycarbonat-Sequenzen.

2. Kosmetische Zusammensetzung nach Anspruch 1, worin das Polyurethan eine Reißdehnung von mindestens 500 % aufweist.

3. Kosmetische Zusammensetzung nach Anspruch 1 oder 2, worin das Polyurethan erhältlich ist unter Verwendung von polymeren Polyether-Polyolen und/oder polymeren Polycarbonat-Polyolen und/oder Polyether-Polycarbonat-Polyolen welche jeweils zahlenmittlere Molekulargewichte von 400 bis 8000 g/mol aufweisen.

4. Kosmetische Zusammensetzung nach einem der Ansprüche 1 bis 3, worin das Polyurethan mindestens eine anionische Gruppe und/oder potentiell anionische Gruppe aufweist.

5. Kosmetische Zusammensetzung nach einem der Ansprüche 1 bis 4, welche 1 bis 90 Gew.-%, bevorzugt mehr als 30 Gew.-% des Polyurethans, bezogen auf den Festkörpergehalt der kosmetischen Zusammensetzung enthält.

6. Kosmetische Zusammensetzung nach einem der Ansprüche 1 bis 5, worin als aminofunktionelle Verbindung in das Polyurethan wenigstens eine aminofunktionelle Verbindung B2) eingebaut ist, die ionische und/oder ionogene Gruppen aufweist, bevorzugt 2-(2-Aminoethylamino)ethansulfonsäure und/oder deren Salze.

7. Kosmetische Zusammensetzung nach einem der Ansprüche 1 bis 6, worin als aminofunktionellen Verbindung B) in das Polyurethan wenigstens eine aminofunktionelle Verbindung B1) eingebaut ist, die keine ionische und/oder ionogene Gruppen aufweist, und bevorzugt ein Diamin isz, dass keine ionischen und/oder ionogenen Gruppen aufweist.

8. Kosmetische Zusammensetzung nach einem der Ansprüche 1 bis 7, welche weniger als 1 Gew.-%, nicht-filmbildender Füllstoffe enthält.

9. Kosmetische Zusammensetzung nach einem der Ansprüche 1 bis 8 zur Herstellung einer Gesichtsmaske.

10. Kosmetische Zusammensetzung nach einem der Ansprüche 1 bis 9, enthaltend mindestens einen kosmetisch und/oder therapeutisch wirksamen Inhaltsstoff.

11. Verwendung einer kosmetischen Zusammensetzung nach einem der Ansprüche 1 bis 10 zum Auftrag auf die Haut, bevorzugt auf die Gesichtshaut.

12. Kosmetisches Verfahren zur Reinigung und/oder Pflege der Haut, welches den Auftrag der Zusammensetzung nach einem der Ansprüche 1 bis 10 auf die Haut, bevorzugt die Gesichtshaut, und das anschließende Entfernen der Zusammensetzung von der Haut, bevorzugt der Gesichtshaut, umfasst.

13. Verwendung eines Polyurethans, welches eine Reißdehnung von mindestens 500 % aufweist, zur Herstellung kosmetischer Zusammensetzungen zum Auftrag auf die Haut, bevorzugt die Gesichtshaut.
